(19) Europäisches Patentamt
European Patent Office
Office européen des brevets

(11) Publication number: **0 134 096**
**B1**

(12) # EUROPEAN PATENT SPECIFICATION

(45) Date of publication of patent specification: **01.02.89**

(21) Application number: **84305045.1**

(22) Date of filing: **25.07.84**

(51) Int. Cl.$^4$: **C 07 D 239/46,**
C 07 D 239/48,
C 07 D 401/12,
C 07 D 401/14,
C 07 D 403/12,
C 07 D 405/12,
C 07 D 409/12,
C 07 D 417/12, A 61 K 31/505

(54) **Aminopyrimidinone derivatives as histamine H2-antagonists.**

(30) Priority: **29.07.83 GB 8320505**

(43) Date of publication of application:
**13.03.85 Bulletin 85/11**

(45) Publication of the grant of the patent:
**01.02.89 Bulletin 89/05**

(84) Designated Contracting States:
**AT BE CH DE FR GB IT LI LU NL SE**

(56) References cited:
**EP-A-0 003 677**
**EP-A-0 013 071**
**EP-A-0 015 138**
**EP-A-0 049 173**
**EP-A-0 083 186**
**GB-A-2 030 979**

**The file contains technical information submitted after the application was filed and not included in this specification**

(73) Proprietor: **SMITH KLINE & FRENCH LABORATORIES LIMITED**
**Mundells**
**Welwyn Garden City Hertfordshire, AL7 1EY (GB)**

(72) Inventor: **Brown, Thomas Henry**
**17 Godfries Close**
**Tewin Hertfordshire (GB)**
Inventor: **Tuddenham, David**
**Flat 4 One Hallplace Gardens**
**St Albans Hertfordshire (GB)**

(74) Representative: **Denerley, Paul Millington, Dr. et al**
**Smith Kline & French Laboratories Ltd Patent Department Mundells**
**Welwyn Garden City Hertfordshire AL7 1EY (GB)**

**EP 0 134 096 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to aminopyrimidinone derivatives, pharmaceutical compositions containing them, their use as histamine $H_2$-antagonists, processes for their preparation and intermediates therefor.

Histamine, a physiologically active compound endogenous in mammals, exerts its action by interacting with certain sites called receptors. One type of receptor is known as a histamine $H_1$-receptor (Ash and Schild, Brit. J. Pharmac. Chemother. *27* 427 (1966)) and the actions of histamine mediated through these receptors are blocked by drugs commonly called "antihistamines" (histamine $H_1$-antagonists) a common example of which is mepyramine. A second type of histamine receptor is known as the $H_2$-receptor (Black et al. Nature 1972, *236,* 385). These receptors are not blocked by mepyramine but are blocked by burimamide. Compounds which block these histamine $H_2$-receptors are called histamine $H_2$-antagonists.

Histamine $H_2$-antagonists are useful in treating disease conditions caused by the biological effects of histamine mediated through $H_2$-receptors, for example, as inhibitors of gastric acid secretion, in the treatment of inflammation mediated through histamine $H_2$-receptors and as agents which act on the cardiovascular system, for example, as inhibitors of effects of histamine on blood pessure mediated through histamine $H_2$-receptors.

Cimetidine is an example of a histamine $H_2$-antagonist. Cimetidine has been shown to be useful in the treatment of duodenal, gastric, recurrent and stomal ulceration, and reflux oesophagitis and in the management of patients who are at high risk from haemorrhage of the upper gastrointestinal tract.

In some physiological conditions the biological actions of histamine are mediated through both histamine $H_1$- and $H_2$-receptors and blockade of both types of receptors is useful. These conditions include inflammation mediated by histamine, for example skin inflammation, and those hypersensitivity responses due to the action of histamine at $H_1$- and $H_2$-receptors, for example allergies.

In the histamine $H_2$-antagonist art there have been a large number of applications directed to compounds comprising a pyrimidinone ring substituted in the 5-position. Such applications include British Specifications 1582527, 1595291 and 2030979, and EP-A-3677, 10894, 13071, 15138 and 49173. Despite the interest shown in such compounds there has never been any suggestion of the 5-substituent being an amino or alkanoyl-amino group. Japanese Patent Application Nos 55—115877 and 55—115860 disclose compounds of the formulae:

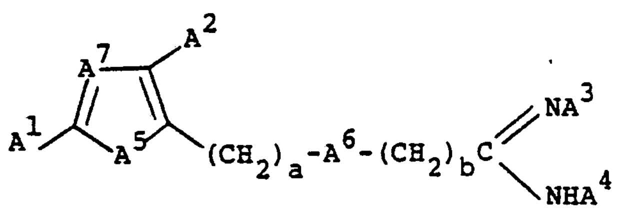

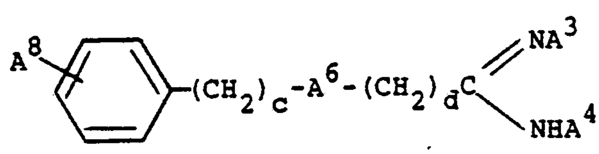

wherein $A^7$ is nitrogen or methine (=CH—); $A^5$ and $A^6$ are the same or different and are oxygen or sulphur; $A^1$ is hydrogen, lower alkyl, substituted or unsubstituted amino(lower)alkyl or cycloalkylamino(lower)alkyl optionally interrupted with oxygen; $A^2$ is hydrogen or lower alkyl; a and b are each 1 to 3; c is 0 to 2; d is 1 to 4; $A^8$ is substituted or unsubstituted amidino, substituted or unsubstituted guanidino, biguanidino, hydrazino or a group of formula:
$R^aR^bN$-Alk- wherein Alk is straight or branched chain lower alkylene and $R^a$ and $R^b$ are the same or different and are hydrogen or lower alkyl or $R^a$ and $R^b$ together with the nitrogen atom may form a heterocyclic group optionally containing an oxygen atom; $A^3$ and $A^4$ represent a wide variety of substituents and *inter alia* can together with the carbon atom and the nitrogen atoms bonded thereto form an optionally substituted 5- or 6-membered heterocyclic group. However there is no suggestion in either application of amino or alkanoylamino being a substituent for any such heterocyclic ring, and no suggestion that pyrimidinone is a favoured ring system.

A small group of compound has now been invented, which have a 5-amino or 5-alkanoylamino substituent on a pyrimidin-4-one ring and have a particularly favourable level of activity and duration as $H_2$-antagonists.

Accordingly the present invention provides a compound of the formula (I):

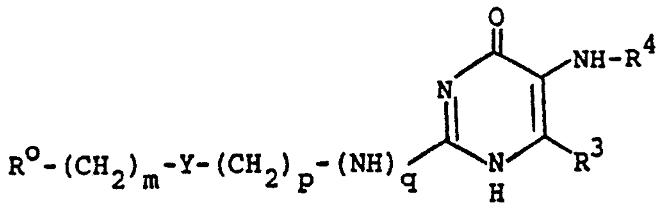

(I)

or a salt thereof, wherein:

$R^0$ is 2-guanidinothiazol-4-yl or a group $R^1R^2N(CH_2)_n$—Z— wherein:

$R^1$ and $R^2$ are independently hydrogen, $C_{1-6}$alkyl, benzyl, phenethyl, furanyl($C_{1-6}$)alkyl, thienyl($C_{1-6}$)alkyl, $C_{3-10}$cycloalkyl, hydroxy($C_{2-6}$)alkyl, or halo ($C_{1-6}$)alkyl (wherein said hydroxy and halo groups are not substituted on the carbon atom adjacent to the nitrogen atom); or

$R^1$ or $R^2$ together represent —$(CH_2)_r$— wherein r is 4 to 7, to form together with the nitrogen atom to which they are attenched a 5—8 membered saturated ring;

n is an integer from 1 to 6;

Z is 2,5-furanyl, 2,5-thienyl, 2,4-pyridyl wherein the $R^1R^2N(CH_2)_n$ group is in the 4-position, 2,4-thiazolyl wherein the $R^1R^2N(CH_2)_n$ group is in the 2-position, or 1,3- or 1,4-phenylene;

m is one; or if Z is pyridyl or phenylene m may also be zero;

Y is oxygen, sulphur or methylene; or if Z is furanyl, thienyl or thiazolyl Y may also be a bond;

p is two, three or four;

q is zero or one;

$R^3$ is hydrogen or $C_{1-6}$alkyl; and

$R^4$ is hydrogen or $C_{1-6}$alkanoyl.

The compounds of this invention are preferably provided in a form suitable for pharmaceutical use as a compound of the formula (I) or a pharmaceutically acceptable salt thereof.

When used herein 'alkyl' means groups that are either straight-chained or branched. In general preferred alkyl groups are methyl and ethyl.

In one aspect of this invention $R^0$ is a group $R^1R^2N(CH_2)_n$—Z— as herein defined.

Suitably $R^1$ is benzyl, phenethyl, furanyl($C_{1-6}$)alkyl such as furanylmethyl, thienyl($C_{1-6}$)alkyl such as thienylmethyl, halo($C_{2-6}$)alkyl for example 2,2,2-trifluoroethyl, or $C_{3-10}$cycloalkyl for example cyclohexyl. More suitably $R^1$ is $C_{1-6}$alkyl, for example methyl, ethyl or propyl.

Suitably $R^2$ is hydrogen or $C_{1-6}$alkyl, for example methyl, ethyl or propyl.

Suitably $R^1$ and $R^2$ have the same value, for example they both are methyl or they are both ethyl. In another suitable aspect $R^1$ and $R^2$ together with the nitrogen atom to which they are attached form a pyrrolidino, piperidino or hexahydroazepino ring, preferably a piperidino ring.

Preferably n is one.

Suitably Z is 2,5-furanyl or 2,5-thienyl. In such compounds preferably Y is sulphur. For example $R^1R^2N(CH_2)_n$—Z—$(CH_2)_m$—Y may represent 5-dimethylaminomethylfuran-2-ylmethylthio, 5-piperidinomethylfuran-2-ylmethylthio or 5-pyrrolidinomethylfuran-2-ylmethylthio.

In another aspect Z is 2,4-thiazolyl. In such compounds preferably Y is sulphur, for example $R^1R^2N(CH_2)_n$—Z—$(CH_2)_m$—Y— may represent 2-dimethylaminomethylthiazol-4-ylmethylthio. In a further aspect $R^0$ is 2-guanidinothiazol-4-yl, in such compounds preferably Y is sulphur, for example $R^0$—$(CH_2)_m$—Y— may represent 2-guanidinothiazol-4-ylmethylthio.

In a preferred aspect Z is 2,4-pyridyl. In an alternative preferred aspect Z is 1,3-phenylene. In each type of compound preferably —$(CH_2)_m$Y—$(CH_2)_p$— is —O—$(CH_2)_3$— or —$CH_2SCH_2CH_2$—. For example $R^1R^2N(CH_2)_n$—Z—$(CH_2)_m$—Y—$(CH_2)_p$— may represent:

4-dimethylaminomethylpyrid-2-ylmethylthioethyl,
4-piperidinomethylpyrid-2-ylmethylthioethyl,
4-dimethylaminomethylpyrid-2-yloxypropyl,
4-piperidinomethylpyrid-2-yloxypropyl,
3-dimethylaminomethylphenoxypropyl,
3-piperidinomethylphenoxypropyl,
3-dimethylaminomethylphenylmethylthioethyl or
3-piperidinomethylphenylmethylthioethyl.

Preferably p is 3 when m is zero. Preferably p is 2 when m is one.

Suitably q is zero. Suitably q is one.

Suitably $R^3$ is $C_{1-6}$alkyl for example methyl. Preferably $R^3$ is hydrogen.

Suitably $R^4$ is $C_{1-6}$alkanoyl for example formyl, acetyl or propionyl. Preferably $R^4$ is hydrogen.

Suitably $R^1R^2N(CH_2)_n$—Z— is 3-piperidinomethylphenyl.

In a favoured aspect the present invention provides compounds of the formula (I) wherein $R^1R^2N(CH_2)_n$— is dimethylaminomethyl or piperidinomethyl, Z is 1,3-phenylene, m is zero, Y is oxygen, p is 3, q is zero or one, and $R^3$ and $R^4$ are both hydrogen.

The compounds of the formula (I), are shown and described as 4-pyrimidinone derivatives and these derivatives exist in equilibrium with the corresponding 6-pyrimidinone tautomers. These compounds also

exist to a lesser extent as the hydroxy tautomers and the pyrimidinone ring, when q is one, may also exist in the following tautomeric forms:

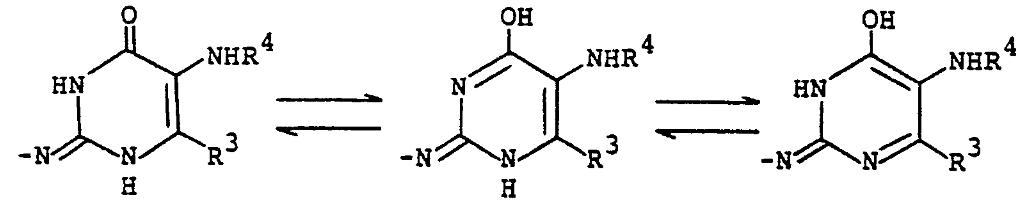

The activity of the compounds of formula (I) as histamine $H_2$-antagonists can be demonstrated by their ability to inhibit histamine-stimulated secretion of gastric acid from the lumen-perfused stomachs of rats anaesthetised with urethane, and to reverse histamine-induced inhibition of contractions of the isolated rat uterus. These are actions of histamine which, according to Ash and Schild, Brit. J. Pharmac. Chemother. *27,* 247 (1966), are not mediated by histamine $H_1$-receptors.

The histamine $H_2$-antagonist activity of the compounds can also be demonstrated by the inhibition of histamine-stimulated acid secretion in the Heidenhain Pouch Dog, the inhibition of histamine-induced tachycardia in the isolated guinea pig right atrium and the inhibition of histamine-induced vasodilatation in the anaesthetised cat.

The measurement of inhibition of histamine-stimulated secretion of gastric acid from the lumen-perfused stomachs of rats anaesthetised with urethane, and the measurement of inhibition of histamine-induced tachycardia in the isolated guinea pig right atrium, are detailed in European Patent Application Publication No 0049173.

To illustrate the level of activity of the compounds of the invention we have determined that the products of the Examples, where tested, have $ED_{50}$ values in the lumen-perfused rat test of less than 0.1 micromol $kg^{-1}$ i.v. and $pA_2$ values in the guinea pig atrium test of more than six. The product of Example 2 also showed a longer duration of activity than cimetidine after intravenous administration in the Heidenhain pouch dog when dose levels had been adjusted to produce similar peak responses. In addition this compound showed a significant increase in activity compared to a related compound wherein $NHR^4$ is replaced by H, i.e. 2-[3-(3-(piperidinomethyl)methyl)phenoxy)propyl]pyrimidin-4-one.

In order to use the compounds of the formula (I) or pharmaceutically acceptable salts thereof for medical purposes, they are normally formulated in accordance with standard pharmaceutical practice as pharmaceutical compositions.

The invention further provides pharmaceutical compositions comprising a compound of the formula (I) or a pharmaceutically acceptable salt thereof and a pharmaceutically acceptable carrier.

The compounds of the formula (I) and their pharmaceutically acceptable salts may be administered, for example, orally, parenterally, cutaneously or rectally.

The compounds of the formula (I) and their pharmaceutically acceptable salts which are active when given orally can be formulated as syrups, tablets, capsules and lozenges. A syrup formulation will generally consist of a suspension or solution of the compound or pharaceutically acceptable salt in a suitable liquid carrier for example, ethanol, glycerine or water with a flavouring or colouring agent. Where the composition is in the form of a tablet, any suitable pharmaceutical carrier routinely used for preparing solid formulations may be used. Examples of such carriers include magnesium stearate, starch, lactose, sucrose and cellulose.

Typical parenteral compositions consist of a solution or suspension of the compound or pharmaceutically acceptable salt in a sterile aqueous carrier or parenterally acceptable oil.

Typical compositions for administration to the skin include lotions and creams in which the compound of the formula (I) or pharmaceutically acceptable salt thereof is contained in a liquid vehicle.

A typical suppository formulation comprises a compound of formula (I) or a pharmaceutically acceptable salt thereof which is active when adminstered in this way, with a binding and/or lubricating agent such as gelatin or cocoa butter or other low melting vegetable waxes or fats.

Preferably the composition is in unit dose form such as a tablet or capsule.

Each dosage unit for oral administration contains preferably from 15 to 250 mg (and for parenteral administration contains preferably from 0.5 to 25 mg) of a compound of the formula (I) or a pharmaceutically acceptable salt thereof calculated as the free base.

The invention also provides a method of blocking histamine $H_2$ receptors which comprises administering to an animal an effective amount to block said receptors of a compound of the formula (I) or a pharmaceutically acceptable salt thereof.

The pharmaceutically acceptable compounds of the invention will normally be administered to a subject for the treatment of peptic ulcers and other conditions caused or exacerbated by gastric acidity in the same general manner as that employed for known histamine $H_2$-antagonists, due allowance being made in terms of dose levels for the potency of the compound of the present invention relative to known histamine $H_2$-antagonists. The daily dosage regimen for example for an adult patient may be an oral dose

of between 15 mg and 1000 mg, preferably between 20 mg and 250 mg, or an intravenous, subcutaneous, or intramuscular dose of between 0.5 mg and 100 mg, preferably between 1 mg and 20 mg, of the compound of the formula (I) or a pharmaceutically acceptable salt thereof calculated as the free base, the compound being administered 1 to 4 times per day.

In a further aspect of this invention the compounds of the formula (I) and salts thereof may be prepared by a process which comprises:

a) for compounds of the formula (I) wherein q is one, reacting a compound of the formula (II) with a compound of the formula (III):

$R^6(CH_2)_m-Y-(CH_2)_pNH_2$

(II)

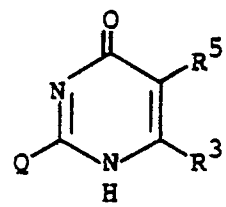

(III)

wherein m, Y, p and $R^3$ are as herinbefore defined, $R^6$ is a group $R^0$ as hereinbefore defined or $R^6$ is a furan-2-yl or thien-2-yl group; $R^5$ is protected amino; and Q is a group displaceable by amine; or

b) for compounds of the formula (I) wherein m is one and Y is sulphur, reacting a compound of the formula (IV) with a compound of the formula (V):

$R^6-CH_2-L$

(IV)

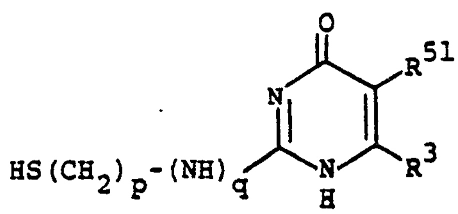

(V)

wherein $R^6$, p, q and $R^3$ are as hereinbefore defined, $R^{51}$ is optionally protected amino and L is a moiety displaceable by thiol or chemical equivalent thereof; or

c) for compounds of the formula (I) wherein m is one and Y is sulphur, reacting a compound of the formula (VI) or chemical equivalent thereof with a compound of the formula (VII):

$R^6CH_2SH$

(VI)

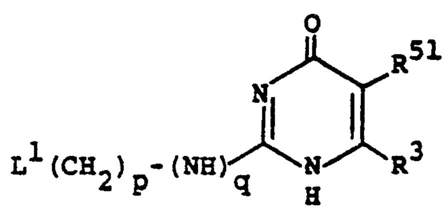

(VII)

wherein $R^6$, p, q, $R^{51}$ and $R^3$ are as hereinbefore defined and $L^1$ is a moiety displaceable by thiol or chemical equivalent thereof; or

d) reacting a compound of the formula (VIII) with a compound of the formula (IX) or chemical equivalent thereof:

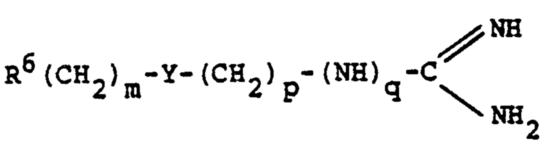

(VIII)

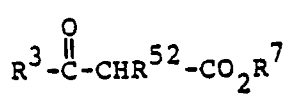

(IX)

wherein $R^6$, m, Y, p, q, and $R^3$ are as hereinbefore defined, $R^{52}$ is protected amino and $R^7$ is an ester-forming group; or

e) for compounds of the formula (I) wherein Z is 2,4-pyridyl, m is zero and Y is oxygen, reacting a

compound of the formula (X) with a compound of the formula (XI) or derivative thereof that permits reaction to occur:

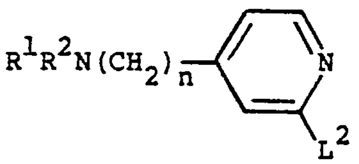

(X)

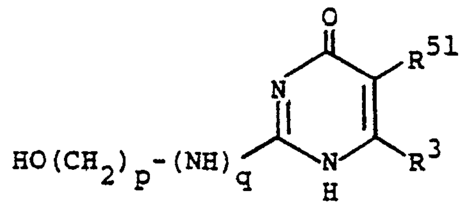

(XI)

wherein $R^1$, $R^2$, n, p, q, $R^3$ and $R^{51}$ are as hereinbefore defined and $L^2$ is a group displaceable by hydroxy or the equivalent thereof; or

f) for compounds of the formula (I) wherein Z is phenylene, m is zero and Y is oxygen, reacting a compound of the formula (XII) or chemical equivalent thereof with a compound of the formula (XIII):

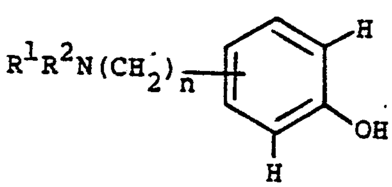

(XII)

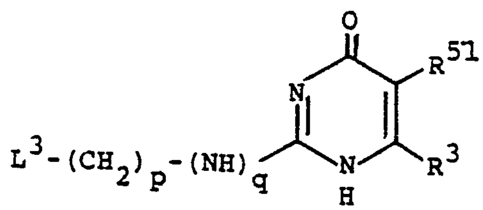

(XIII)

wherein $R^1$, $R^2$, n, p, q, $R^3$ and $R^{51}$ are as hereinbefore defined and $L^3$ is a moiety displaceable by phenol or chemical equivalent thereof; or

g) for compounds of the formula (I) wherein $R^0$ is $R^1R^2N(CH_2)_n$—Z—, converting a compound of the formula (XIV):

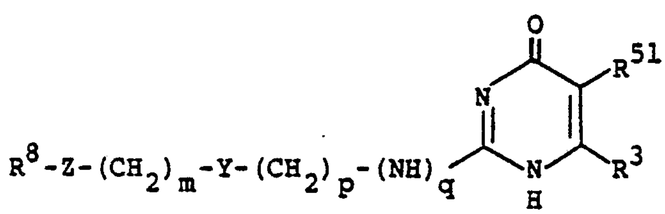

(XIV)

wherein Z, m, Y, p, q, $R^3$ and $R^{51}$ are as hereinbefore defined and $R^8$ is a precursor of a group $R^1R^2N(CH_2)_n$— as hereinbefore defined; or

h) for compounds of the formula (I) wherein q is one, reducing a compound of the formula (XV):

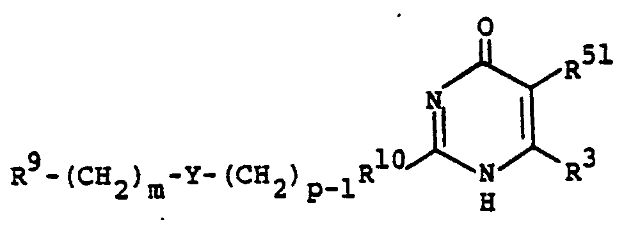

(XV)

wherein m, Y, p, $R^3$ and $R^{51}$ are as hereinbefore defined, $R^9$ is a group $R^0$ or $R^8$—Z— as hereinbefore defined and $R^{10}$ is a group —CH=N— or —CO—NH—;

and thereafter where necessary:

i) reacting a compound wherein $R^6$ is furan-2-yl or thien-2-yl with a Mannich reagent to form a compound of the formula (I) wherein n is one;

ii) converting a protected amino group to amino or $C_{1-6}$alkanoylamino;

iii) optionally forming a salt.

Suitably Q is nitroamino, $C_{1-6}$alkylthio, benzylthio, chloro or bromo. Of these methylthio is preferred.

The reaction between a compound of the formula (II) and a compound of formula (III) can be performed, at an elevated temperature, in the absence of solvent or in the presence of a substantially inert polar solvent. When Q is methylthio the reaction may be performed for example in the absence of solvent at 140°—170°C, or the reaction may be performed in a substantially inert solvent under reflux conditions, for example in a $C_{1-6}$alkanol, pyridine or anisole.

Example of the moiety L include chloro, bromo, hydroxy, $C_{1-6}$alkoxy for example methoxy, $C_{1-6}$alkanoyloxy for example acetoxy, arylsulphonyloxy for example 4-methylbenzenesulphonyloxy, or $C_{1-6}$alkylsulphonyloxy for example methanesulphonyloxy.

Preferably L is hydroxy in which case the reaction between the compounds of the formulae (IV) and (V) is performed under acidic conditions. When L is chloro or bromo it is preferably to perform the reaction in the presence of a strong base for example sodium ethoxide in ethanol. When L is an arylsulphonyloxy or alkylsulphonyloxy group the reaction is preferably performed under mildly basic conditions for example in pyridine solution.

In the reaction of compounds of the formulae (IV) and (V), when carried out under basic conditions about one equivalent of base is used in order that a thiolate anion is preferentially formed on the $HS(CH_2)_q$— moiety.

Suitably in the reaction of compound of the formulae (VI) and (VII) $L^1$ is chloro, bromo, arylsulphonyloxy for example 4-methylbenzenesulphonyloxy or $C_{1-6}$alkylsulphonyloxy for example methylsulphonyloxy. Such reactions are generally performed in the presence of a base for example triethylamine, an alkoxide or a hydroxide.

The compound of the formula (IX) is depicted as a ketone or aldehyde, dependent on whether $R^3$ is alkyl or hydrogen. This invention covers the reaction of chemical equivalents of such depicted compounds, as known in the art, for example protected or 'masked' aldehydes and ketones, for example an acetal.

When it is desired to form a compound of the formula (I) wherein $R^3$ is hydrogen, then for example a compound of the formula (VIII) may be reacted with a compound of the formula (IXA) and thereafter deprotecting if necessary:

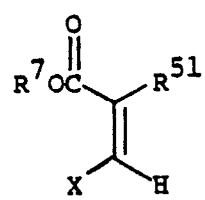

(IXA)

wherein $R^7$ and $R^{51}$ are as hereinbefore defined and X is a displaceable group. For example X may be hydroxy (in which case the form depicted is tautomeric with formula (IX)) or a derivative thereof, so that for example X is protected hydroxy such as silyloxy, an acid residue RCO—O— (for example $C_{1-6}$alkanoyloxy), or an ether forming residue (for example $C_{1-6}$alkoxy such as methoxy or ethoxy). Additional examples for X include secondary and tertiary amino groups, for example di-$C_{1-6}$alkylamino such as dimethylamino, cyclic amines such as piperidino, pyrrolidino and morpholino, anilino and 1-imidazolyl. Preferably X is $C_{1-6}$alkoxy and in particular ethoxy.

In compounds of the formulae (IX) and (IXA) the ester-forming group $R^7$ can be a $C_{1-4}$alkyl group and is preferably methyl or ethyl.

The reaction between the compounds of formulae (VIII) and (IX) or (IXA) is carried out in the presence of base. Examples of suitable bases include alkali metal hydroxides and $C_{1-4}$alkoxides, sodium hydride, and quaternary ammonium hydroxides, for example benzyltrimethylammonium hydroxide. Preferably the base is sodium ethoxide or sodium methoxide. The reaction can be carried out in the presence of a solvent the choice of which is not critical to the success of the process provided that it is substantially inert to the reagents and product. Preferably the solvent is a $C_{1-4}$alkanol, (for example, methanol, ethanol or propanol) or dimethylformamide. The reaction can be carried out at moderate temperatures, for example from room temperature to the reflux temperature of the solvent.

Suitably in the compounds of the formula (X), $L^2$ is chloro or bromo. The reaction of a compound of the formula (X) with a compound of the formula (XI) is generally performed under basic conditions, for example the anion of the compounds of the formula (XI) may be generated, for example using sodium hydride in a suitable solvent.

In the reaction between the compounds of the formulae (XII) and (XIII) suitably $L^3$ is chloro or bromo. Suitably the reaction is performed under basic conditions, for example the anion of the compound of the formula (XII) may be generated, for example using sodium hydride. The reaction is performed in a suitable aprotic solvent for example dimethylformamide at a non-extreme temperature for example between 0°C and 100°C, suitably between ambient and 70°C.

In the compounds of the formulae (XIV) and (XV) in one suitable aspect $R^8$ is a group $R^1R^2N(CH_2)_xCO(CH_2)_y$— wherein X+y=n−1. Fabourably x and y are both zero so that the group $R^1R^2NCO$— is a precursor to the group $R^1R^2NCH_2$—. The conversion of such a group $R^1R^2N(CH_2)_xCO(CH_2)_y$— may be performed by reduction for example with a hydride for example lithium aluminium hydride.

In an alternative aspect $R^8$ is a group $CHO—(CH_2)_{n-1}—$ which may be converted to a group $R^1R^2N(CH_2)_n—$ on reaction with an amine $R^1R^2NH$ under conditions of reductive amination. Furthermore in another suitable aspect $R^8$ may be a group $HO(CH_2)_n—$ which may be converted directly to $R^1R^2N(CH_2)_n—$ or indirectly thereto for example via a moiety such as $Br(CH_2)_n—$ and thence to $R^1R^2N(CH_2)_n—$. Such transformations may be carried out in conventional manner.

The compounds of the formula (XV) may be reduced to form compounds of the formula (I) wherein q is one, for example using lithium aluminium hydride in an ether solvent when $R^{10}$ is —CONH—; and for example using a borohydride in an alkanol, lithium aluminium hydride in an ether solvent, or catalytically hydrogenating when $R^{10}$ is —CH=N—.

A suitable protected amino group is nitro ($—NO_2$) which may be converted to amino ($—NH_2$) by reduction. Such nitro compounds are primarily of interest as intermediates, but also have $H_2$-antagonist activity in their own right.

One suitable method of reduction of the nitro compounds comprises catalytic hydrogenation in the presence of a suitable transition metal catalyst for example palladium or platinum. In one aspect hydrogen gas may be used over a palladium catalyst, suitably palladium on carbon, for example 10% palladium on carbon, in a suitable solvent such as $C_{1-6}$alkanol, in particular methanol or ethanol. In another aspect catalytic transfer hydrogenation may be employed, for example using cyclohexene and palladium on carbon, conveniently in a mixed solvent system, for example with a $C_{1-6}$alkanol such as methanol. Such catalytic transfer hydrogenation is normally performed at elevated temperatures, conveniently under conditions of reflux. In yet another suitable aspect a transition metal catalyst for example palladium on carbon or Raney nickel may be used in the presence of hydrazine (conveniently as the hydrate), suitably at elevated temperatures for example under reflux, in a $C_{1-6}$alkanol for example ethanol. Suitably the nitro group may be converted to amino with a suitable anionic sulphur compound. For example a dithionite salt, conveniently the sodium salt may be used in the presence of aqueous ammonia at a basic pH.

In a preferred aspect of this invention it has been found that unexpectedly good yields of a compound of the formula (I) wherein $R^4$ is amino are obtained from a corresponding nitro compound using stannous chloride under substantially neutral conditions. Conveniently stannous chloride is in anhydrous form or in the form of the dihydrate. The reaction is preferably performed at a non-extreme temperature, for example between 0°C and 50°C, favourably at ambient temperature. The reaction is preferably performed in a $C_{1-6}$alkanol, in particular ethanol, or in ethyl acetate. Mixtures of these solvents may also be used.

Other suitable methods of reducing the nitro group to the amino group include using suitable metals in the presence of acid, for example iron and hydrochloric acid or zinc and hydrochloric acid. In addition hydride reductions may be employed, for example using lithium aluminium hydride or sodium borohydride.

Accordingly in another aspect of this invention there is provided a process for preparing a compound of the formula (I) wherein $R^4$ is hydrogen which comprises reducing a compound of the formula (XVI):

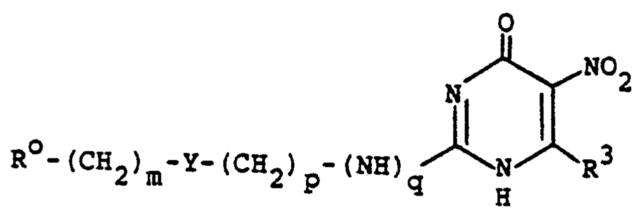

(XVI)

wherein $R^0$, m, Y, p, q and $R^3$ are as hereinbefore defined.

Another suitable protected amino group is optionally substituted benzylidene, for example formed by reaction of an amino group ($-NH_2$) with benzaldehyde. This protecting group is cleavable on treatment with mild acid.

Other suitable protected amino groups include tertiary-butyloxycarbonylamino removable by trifluoroacetic acid, benzyloxycarbonylamino removable by hydrogenolysis or hydrobromic acid, and p-nitrobenzyloxycarbonylamino removable by hydrogenolysis.

In addition the term protected amino covers those compounds wherein $R^5$ is $C_{1-6}$alkanoylamino, thus leading directly to compounds within formula (I).

Furthermore compounds of the formula (I) wherein $R^4$ is $C_{1-6}$alkanoylamino may be prepared from compounds of the formula (I) wherein $R^4$ is amino by conventional methods of acylation for example reaction with an acid halide, in particular a chloride, in an organic solvent. In an alternative a compound of the formula (I) wherein $R^4$ is $C_{1-6}$alkanoylamino may be prepared from a corresponding nitro compound by dissolving metal reduction for example with iron powder and $C_{1-6}$alkanoic acid.

For converting a compound wherein $R^6$ is furan-2-yl or thien-2-yl to a compound of the formula (I) wherein m is 1, suitable Mannich reagents include formaldehyde and an amine $R^1R^2NH$ or salt thereof. Such a reaction may be carried out by treatment of an amine salt with aqueous formaldehyde and a compound wherein $R^6$ is unsubstituted furan-2-yl or thien-2-yl, or by heating an amine salt with

paraformaldehyde and a compound wherein $R^6$ is unsubstituted furan-2-yl or thien-2-yl, in a convenient solvent such as ethanol. Alternatively where $R^1$ and $R^2$ are both $C_{1-4}$alkyl, the Mannich reagent may be a di-($C_{1-4}$alkyl)methylene ammonium salt for example dimethylmethylene ammonium chloride or iodide, or may be a bis di-$C_{1-4}$alkylaminomethane, for example bis(dimethylamino)methane.

Any group in the remainder of the molecule that is capable of reacting with a Mannich reagent may be optionally protected during the reaction, and may be subsequently deprotected in conventional manner. Thus any deprotection of the 5-pyrimidinone substituent is preferably performed after the Mannich reaction.

The formation of the group $R^1R^2N(CH_2)_n$— may be performed at any convenient stage of the synthetic procedures outlined herein or in the art. Such introduction may be direct or may involve two or more steps for example converting a hydroxyalkyl substituent to bromoalkyl and subsequently to $R^1R^2N(CH_2)_n$—.

Pharmaceutically acceptable acid addition salts of the compounds of the formula (I) may be prepared from the corresponding base of the compounds of the formula (I) in conventional manner. For example the base may be reacted with an acid in a $C_{1-4}$alkanol, or an ion-exchange resin may be used. The salts of the compounds of the formula (I) may be interconverted using ion-exchange resins. Non-pharmaceutically acceptable salts are therefore of use as they can be converted to pharmaceutically acceptable salts.

Suitable pharmaceutically acceptable acid addition salts of the compounds of the formula (I) include those formed with hydrochloride, hydrobromic, sulphuric, phosphoric, acetic, citric, maleic, lactic, ascorbic, fumaric, oxalic, methanesulphonic and ethanesulphonic acids.

The compounds of the formula (II), (IV), (VI) and (X) may be prepared for example by the methods described in European Patent Application Publication Nos. 3677, 4793, 13071, 15138, 17679, 17680, 24873 and 49173 and UK Patent Application 2030989A.

The compound of the formula (III) wherein Q is $C_{1-6}$alkylthio or benzylthio may be prepared by the reaction of a compound of the formula (XVII):

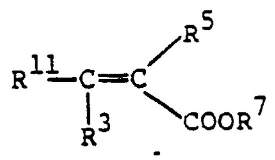

(XVII)

wherein $R^3$, $R^5$ and $R^7$ are as hereinbefore defined, and $R^{11}$ is hydroxy or $C_{1-6}$alkoxy; with thiourea followed by alkylation or benzylation, and subsequently if desired converting one protected amino group, such as nitro, to another protected amino group.

The compounds of the formula (III) wherein Q is chloro or bromo may be prepared by the reaction of a compound of the formula (XVII) with guanidine, followed by diazotisation in hydrochloric acid in the presence of cuprous chloride and copper, or by diazotisation in hydrobromic acid in the presence of cuprous bromide and copper. Subsequently if desired one protected amino group, such as nitro, can be converted to another protected amino group.

Compounds of the formula (III) with other values of Q may be prepared in conventional manner.

Preferably the reactions of the compound of the formula (XVII) with thiourea and guanidine are carried out in the presence of a base, for example, an alkali metal lower alkoxide, preferably sodium methoxide or sodium ethoxide, an alkali metal carbonate or hydroxide, preferably potassium carbonate or sodium hydroxide, sodium hydride or a quaternary ammonium hydroxide, for example benzyltrimethylammonium hydroxide. Preferably this reaction is carried out at an elevated temperature, for example the reflux temperature of the solvent mixture. Preferably the solvent is a lower alkanol, for example ethanol, an aqueous lower alkanol, a ketone, for example 2-butanone, or a polar aprotic solvent, for example dimethylformamide. The compound of the formula (XVII) may also be used in the form of a hemiacetal, for example of a $C_{1-6}$alkanol.

The compounds of the formula (XVII) may be prepared by the methods of Chem Berichte (1973), 106, p 3053. In addition this reference teaches the preparation of 5-nitro-2-thiouracil which may be converted to a compound of the formula (III) wherein $R^5$ is nitro and Q is $C_{1-6}$alkylthio or benzylthio on reaction with an alkylating or benzylating agent.

Compounds of formula (V) wherein q is one may be prepared by the reaction of a corresponding compound of the formula (III) with an aminoalkylthiol or protected derivative thereof. Compounds of the formulae (XI), (VII) or (XIII) wherein q is one may be prepared by the reaction of a corresponding compound of the formula (III) with an aminoalkanol and if necessary subsequently converting a hydroxy group to a group $L^1$ or a group $L^3$.

The compounds of the formula (VIII) can be prepared by reacting a compound of formula (XVIII):

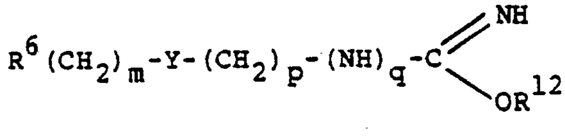

(XVIII)

wherein $R^6$, m, Y, p and q are as hereinbefore defined and $R^{12}$ is a $C_{1-4}$alkyl group in particular methyl, with ammonia or an ammonium salt (for example a halide and particularly the chloride) in the presence of a polar organic solvent, for example a $C_{1-4}$alkanol, in particular methanol, at low to moderate temperatures, for example from 0°C to the reflux temperature of the solvent and in particular at room temperature.

The compounds of the formula (XVIII) wherein q is one are either known or can be prepared in conventional manner, for example by the methods of European Patent Application Publication Nos 49173 and 87274, Belgian Patent 867106 and UK Specification 2030979. The compounds of the formula (XVIII) wherein q is zero are either known or can be prepared in conventional manner, for example by reacting the corresponding nitrile of the formula (XIX):

$$R^6(CH_2)_mY(CH_2)_p—CN \qquad (XIX)$$

wherein $R^6$, m, Y, and p are as hereinbefore defined with the corresponding $C_{1-4}$alkanol under acidic conditions. In particular the alkanol is methanol and the acid is hydrochloric acid.

By way of example, compounds of the formula (XIX) wherein m is zero and Y is oxygen may be prepared by the general method described in Description I hereinafter.

By way of example, compounds of the formula (XIX) wherein $—(CH_2)_mY—$ is $—CH_2S—$ may be prepared by the reaction of a compound of the formula (XX):

$$Hal—(CH_2)_p—CN \qquad (XX)$$

wherein Hal is chlorine, bromine or iodine and p is as hereinbefore defined with a compound of the formula (VI). Preferably Hal is chlorine.

The compound of the formula (VI) may be formed *in situ* from a compound of the formula (XXI):

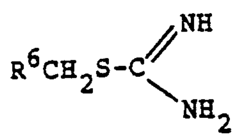

(XXI)

or salt thereof, in particular hydrochloride, in the presence of base for example aqueous sodium hydroxide.

The compounds of the formula (XXI) may be prepared from a compound of the formula (XXII):

$$R^6CH_2Hal^1 \qquad (XXII)$$

wherein $Hal^1$ is chlorine, bromine or iodine, preferably chlorine, with thiourea.

The compounds of the formula (V) wherein q is zero may be prepared by the reaction of a compound of the formula (XXIII):

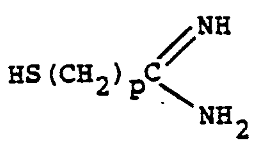

(XXIII)

wherein p is as hereinbefore defined and the thiol moiety is suitably protected, with a compound of the formula (IX) or (IXA), under similar conditions to those described hereinbefore for the reaction with compounds of the formula (VIII). The compounds of the formula (XI), (VII) and (XIII) wherein q is zero may be prepared from conversion of the thiol group of compounds of the formulae (V) or may be prepared by the reaction of a formula (XXIV):

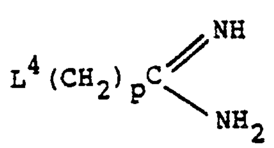

(XXIV)

wherein $L^4$ is hydroxy or a group $L^1$ or $L^3$ as hereinbefore defined and p is as hereinbefore defined, and $L^4$ is suitably protected if desired, with a compound of the formula (IX) or (IXA).

The compounds of the formulae (XXIII) and (XXIV) may be prepared from the corresponding nitriles in an analogous manner to that described above.

The compounds of the formula (XIV) may be prepared in a manner analogous to that described for the preparation of compounds of the formula (I), for example reacting a compound of the formula (III) with an analogue of the formula (II) wherein $R^6$ is replaced by $R^8$, provided that $R^8$ is suitably protected as necessary.

The compounds of the formula (XV) wherein $R^{10}$ is CH=N may be prepared by the reaction of a compound of the formula (XXV) with a compound of the formula (XXVI):

$$R^9(CH_2)_m\text{-}Y\text{-}(CH_2)_{p-1}CHO$$

(XXV)

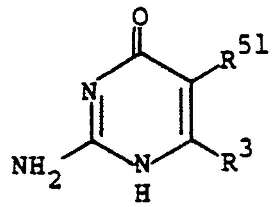

(XXVI)

wherein $R^9$, m, Y, p, $R^3$ and $R^{51}$ are as hereinbefore defined, optionally in the presence of an acid catalyst.

The compounds of the formula (XV) wherein $R^{10}$ is —CONH— may be prepared by the reaction of a compound of the formula (XXVI) with an activated derivative of a compound of the formula (XXVII):

$$R^9\text{—}(CH_2)_m\text{—}Y\text{—}(CH_2)_{p-1}CO_2H \qquad \text{(XXVII)}$$

wherein $R^9$, m, Y, and p are as herinbefore defined. Suitable active derivatives are acyl halides, anhydrides and activated esters. The aldehydes of the formula (XXV) may be prepared for example by reacting a compound of the formula (XII) with a protected bromopropionaldehyde (for example protected as a cyclic acetal) and deprotecting. The acid of the formula (XXVII) and derivatives thereof may be prepared in a similar manner for example by reacting a compound of the formula (XII) with a protected bromopropionic acid and if necessary deprotecting and/or converting to the desired activated acid derivative.

The following Description and Examples serve to illustrate this invention.

Description 1

2-Methylthio-5-nitropyrimidin-4-one

A solution of sodium hydroxide pellets (6.84 g, 0.171 M) in distilled water 200 ml) was added to a suspension of 5-nitro-2-thiouracil (27.41 g, 0.158 M) in ethanol (400 ml). The mixture was warmed on the steam bath to effect solution and iodomethane (22,47 g, 0.158M) added. The resulting solution was heated on the steam bath for one hour and allowed to cool. The solid which crystallised out during heating was filtered off, washed with water and dried *in vacuo* to yield buff crystals (11.91 g), m.p. 208—10°C. The mother liquor afforded further crystals after concentrating and cooling (1.22 g), m.p. 201—5°C.

The two solids were combined and recrystallised from water/glacial acetic acid to give the title compound as pale yellow needles (7.12 g), m.p. 214—16°C.

Description 2

(a) A solution of 3-(piperidinomethyl)phenol (12.05 g) in dry tetrahydrofuran (50 ml) was added dropwise to a suspension of sodium hydride [from 6.0 g of 50% suspension in oil washed with petroleum ether] in dry tetrahydrofuran (70 ml). The mixture was stirred under reflux for 30 minutes and allowed to cool. 4-Bromobutyrolitrile (14.89 g, 10.0 ml) was added to the cool mixture and the mixture so obtained was stirred under reflux for 22 hours. Tetrahydrofuran was evaporated under reduced pressure and the residue was dissolved in water (100 ml). The solution was extracted with ether (3×50 ml), and the aqueous layer was adjusted to pH 9 with $K_2CO_3$. The aqueous solution was extracted with chloroform (3×100 ml), the chloroform extracts were dried ($K_2CO_3$) and chloroform was removed under reduced pressure to give a pale brown oil (16.22 g). This oil was purified by column chromatography (Kieselgel 60 70—230 mesh silica) using chloroform as eluant. The fractions containing the desired product were collected and the solvent was evaporated under reduced pressure to give 4-[3-(piperidinomethyl)phenoxy]butyronitrile as an oil (11.37 g).

(b) Dry halogen chloride gas was introduced into a stirring solution of 4-[3-(piperidinomethyl)-phenoxy]butyronitrile (15.62 g) in dry methanol (65 ml) and dry chloroform (130 ml), cooled to −5°C (ice-salt bath), under nitrogen, over 1.75 hours, at a rate such that the temperature did not exceed 1°C. The solution was stirred at 0°C for a further hour, and left to stand (at about 4°C) for about 16 hours. The mixture was poured on to a solution of potassium carbonate (45 g) in ice-water (500 ml). The organic layer was separated off and the aqueous layer extracted with chloroform (4×150 ml). The combined organic layers were dried ($K_2CO_3$) and evaporated under reduced pressure to give methyl 4-[3-(piperidinomethyl)-phenoxy]butyronimidate (14.46 g) as an oil.

(c) Methyl 4-[3-(piperidinomethyl)phenoxy]butyronimidate (14.46 g), ammonium chloride (2.66 g) and ethanol (100 ml) were stirred at room temperature for 5 hours and allowed to stand for about 16 hours. Ethanol was removed under reduced pressure and the residue purified using medium pressure chromatography, with Kieselgel 60 silica 70—230 mesh (pre-column) and 230—400 mesh (main column) and a

chloroform methanol gradient elution. The fractions containing the desired product (9:1 chloroform:methanol) were collected and the solvent was evaporated under reduced pressure to give 4-[3-(piperidinomethyl)phenoxy]butyronamidine hydrochloride (8.03 g) as a glassy solid.

Example 1

2-[3-(3-(Dimethylaminomethyl)phenoxy)propylamino]-5-aminopyrimidin-4-one

i) 2-[3-(3-(Dimethylaminomethyl)phenoxy)propylamino]-5-nitropyrimidin-4-one

3-[3-(Dimethylaminomethyl)phenoxy)propylamine (2.92 g) and 5-nitro-2-methylthiopyrimidin-4-one (2.25 g) were stirred under reflux in anhydrous pyridine (40 ml) for 5 hours. The solvent was removed under reduced pressure and the semi-solid residue crystallised by trituration under hot propan-2-ol. On cooling the yellow solid was collected by filtration, washed thoroughly with propan-2-ol and dried in vacuo to yield 2-[3-(3-(dimethylaminomethyl)phenoxy)propylamino]-5-nitropyrimidin-4-one (3.37 g), m.p. 211—13°C.

ii) 2-[3-(3-(Dimethylaminomethyl)phenoxy)propylamino]-5-aminopyrimidin-4-one

Part of the product from i) above (3.21 g) was dissolved in a solution of 2N hydrochloride acid (70 ml) and ethanol (50 ml). This solution was hydrogenated for 3 hours over 10% Palladium on carbon (0.25 g) in a 250 ml Parr hydrogenation vessel, at 344 kPa (50 p.s.i.). The reaction mixture was filtered through diatomaceous earth and the filtrate was evaporated under reduced pressure to afford a gel-like solid. Recrystallisation from ethanol, washing the crystals with ether and drying in vacuo afforded the title compound as a solid (2.55 g), m.p. 170—72°C.

This solid was dissolved in water and taken to pH 9.0 with 2N sodium hydroxide solution. An oil precipitated, this was extracted into dichloromethane (four times), these extracts were combined, evaporated under reduced pressure and the residue crystallised from ethanolic HCl to afford a pale yellow solid. Recrystallisation from aqueous methanol afforded the title compound as the trihydrochloride (0.49 g), m.p. 199—201°C.

Example 2

2-[3-(3-(Piperidinomethyl)phenoxy)propyl]-5-aminopyrimidin-4-one

i) 2-[3-(3-(Piperidinomethyl)phenoxy)propyl]-5-nitropyrimidin-4-one

To a solution of sodium metal (0.37 g) in methanol (20 ml) was added 4-[3-(piperidinomethyl)phenoxy)-butyronamidine hydrochloride (1.92 g) with subsequent heating under reflux for 30 minutes. The solution was cooled, ethyl ethoxymethylene nitroacetate (1.16 g) in methanol (10 ml) was added and the resultant mixture stirred under reflux for 5 hours. The mixture was cooled and evaporated under reduced pressure to afford a residue which was dissolved in water (50 ml). This solution was washed with chloroform (3×20 ml), and acidified to pH 9 with glacial acetic acid to afford a precipitated oily solid which was extracted into chloroform containing a small amount of methanol (4×30 ml). The organic extracts were combined, dried ($MgSO_4$) and evaporated under reduced pressure to give a yellow oil which crystallised from ispropanol/methanol/diethylether to give 2-[3-(3-(piperidinomethyl)phenoxy)propyl]-5-nitropyrimidin-4-one as a pale yellow crystalline solid (1.17 g), m.p. 160—162°C.

ii) 2-[3-(3-(Piperidinomethyl)phenoxy)propyl]-5-aminopyrimidin-4-one

2-[3-(3-(Piperidinomethyl)phenoxy)propyl]-5-nitropyrimidin-4-one (1.08 g), 10% Palladium on Carbon (0.1 g) and methanol (80 ml) were placed in a 250 ml Parr hydrogenation vessel. The mixture was hydrogenated at 344 kPa (50 p.s.i.) with hydrogen gas for approximately 5 hours at 40°C. The mixture was filtered through diatomaceous earth, evaporated under reduced pressure and subjected to medium pressure chromatography using acetone/methanol (9:1) as eluant. The fractions containing the major component of the reaction mixture were combined and evaporated under reduced pressure to afford the title compound. This was dissolved in ethanolic HCl and evaporated under reduced pressure to give a residue which was recrystallised from isopropanol to give the dihydrochloride (0.19 g), m.p. 251—2°C.

Example 3

2-[3-(3-(Piperidinomethyl)phenoxy)propylamino]-5-acetamidopyrimidin-4-one

i) 2-[3-(3-(Piperidinomethyl)phenoxy)propylamino]-5-nitropyrimidin-4-one

3-[3-(Piperidinomethyl)phenoxy]propylamine (4.71 g) and 5-nitro-2-methylthiopyrimidin-4-one (3.55 g) were stirred under reflux in pyridine (40 ml) for 4 hours. The reaction mixture was allowed to cool and the solvent evaporated under reduced pressure to afford an oily residue. This was recrystallised from 2N hydrochloric acid to give as a pale yellow crystalline solid, 2-[3-(3-(piperidinomethyl)-phenoxy)propylamino]-5-nitropyrimidin-4-one as a hydrochloride salt (6.44 g), m.p. 134—6°C.

ii) 2-[3-(3-(Piperidinomethyl)phenoxy)propylamino]-5-acetamidopyrimidin-4-one

The product from Example 3 i) (added to material from another run) (8.74 g) and iron powder (9.00 g) were refluxed in glacial acetic acid (100 ml) for 4 hours. The reaction mixture was allowed to cool and evaporated under reduced pressure to afford a residue. This residue was dissolved in water, taken to pH 9.5 with aqueous sodium hydroxide, filtered through diatomaceous earth, and the filtrate was extracted with chloroform in a continuous liquid-liquid extractor for 3 hours. The combined chloroform extracts were

dried ($MgSO_4$), evaporated in vacuo and the solid residue crystallised from ispropanol/methanol. The resultant crystals were subjected to chromatography on silica gel using chloroform/methanol/0.880 ammonia (90:10:1) as eluant. The desired fractions were collected, evaporated under reduced pressure and the solid residue recrystallised from ethanol to afford the title compound (1.05 g), m.p. 185—6°C.

Example 4

2-[3-(3-(Piperidinomethyl)phenoxy)propyl]-5-aminopyrimidin-4-one

2-[3-(3-(Piperidinomethyl)phenoxy)propyl]-5-nitropyrimidin-4-one (5.79 g) was dissolved with warming in a mixture of methanol (350 ml) and cyclohexane (150 ml). To the stirred solution, under nitrogen, was added a slurry of 10% palladium on charcoal (approx. 2.0 g) in methanol (10 ml) and the resultant mixture was stirred under reflux for 21 hours.

The mixture was allowed to cool, filtered through diatomaceous earth and the filtrate was evaporated under reduced pressure to afford a residue. The residue was dissolved in methanol, treated with charcoal, evaporated under reduced pressure and subjected to medium pressure chromatography using acetone/methanol (9:1) as eluant. The desired fractions were collected, evaporated under reduced pressure to give the title compound which was treated with dilute ethanolic HCl. The solvent was removed under reduced pressure and the residue crystallised from isopropanol to give the title compound as the dihydrochloride (1.06 g). Recrystallisation from isopropanol-methanol gave material consistent with that of Example 2.

Example 5

2-[3-(3-(Piperidinomethyl)phenoxy)propylamino]-5-aminopyrimidin-4-one

i) 2-[3-(3-(Piperidinomethyl)phenoxy)propylamino]-5-nitropyrimidin-4-one

3-(3-(Piperidinomethyl)phenoxy)propylamine (2.48 g) and 2-methylthio-5-nitropyrimidin-4-one (1.87 g) were dissolved in pyridine (20 ml) and the solution was stirred under reflux for 4 hours. The solution was allowed to cool and evaporated under reduced pressure to give a residue that was washed with diethyl ether and crystallised from ethanol-methanol to give 2-[3-(3-piperidinomethyl)phenoxy)propylamino]-5-nitropyrimidin-4-one as a pale yellow solid (3.35 g), m.p. 149.5°—153°C.

ii) 2-[3-(3-(Piperidinomethyl)phenoxy)propylamino]-5-aminopyrimidin-4-one

2-[3-(3-(Piperidinomethyl)phenoxy)propylamino]-5-nitropyrimidin-4-one (2.0 g) was suspended in water (40 ml) and 25% ammonia solution (approx. 1 ml) was added to take the pH to about 11. To the stirred suspension was slowly added sodium dithionite (4.0 g) whereupon the pH fell to about 9. The mixture was stirred for 4 hours maintaining the pH at 8.5—9 with ammonia solution and then was allowed to stand overnight. Thin layer chromatography indicated some starting material was still present so further sodium dithionite (4 g and another 4 g a few hours later) was added whilst maintaining the pH at 8.5—9.

The mixture was extracted into chloroform (4×50 ml). The combined chloroform extracts were washed with water, dried ($MgSO_4$) and evaporated under reduced pressure to afford a residue. This residue was dissolved in dilute ethanolic NCl, the resultant solution was evaporated under reduced pressure and the residue was dissolved in water (30 ml). On standing starting-material as the hydrochloride came out of solution as a yellow solid (0.69), m.p. 80—83°C. The mother liquor was evaporated to a third of its volume whereupon further starting material came out of solution. The filtrate was evaporated under reduced pressure and the residue recrystallised from ethanol to give the hydrochloride of the title compound as a yellow solid (0.77 g), m.p. 179.5°—181.5°C.

Example 6

2-[3-(4-(Piperidinomethyl)pyrid-2-oxy)propylamino]-5-aminopyrimidin-4-one

i) 2-[3-(4-(Piperidinomethyl)pyrid-2-oxy)propylamino]-5-nitropyrimidin-4-one

3-(4-(Piperidinomethyl)pyrid-2-oxy)propylamine (6.84 g) and 2-methylthio-5-nitropyrimidin-4-one (2.57 g) were dissolved in pyridine (40 ml) and stirred under reflux for 4 hours. The solution was allowed to cool and evaporated under reduced pressure to give a residue that was washed with diethyl ether and crystallised from ethanol-methanol to give 2-[3-(4-(piperidinomethyl)pyrid-2-oxy)propylamino]-5-nitropyrimidin-4-one (3.16 g) as a pale yellow solid, m.p. 86.5°—90°C.

ii) 2-[3-(4-(Piperidinomethyl)pyrid-2-oxy)propylamino]-5-aminopyrimidin-4-one

2-[3-(4-(Piperidinomethyl)pyrid-2-oxy)propylamino]-5-nitropyrimidin-4-one (0.97 g) and stannous chloride dihydrate (2.82 g) were stirred in ethanol (25 ml), under nitrogen, at room temperature, for 20 hours. The majority of the ethanol was evaporated under reduced pressure and ice-water was added. Aqueous sodium bicarbonate was added to pH 8 and the solution was filtered through diatomaceous earth. The filtrate was extracted into chloroform (4×50 ml). The combined extracts were dried ($MgSO_4$) and evaporated under reduced pressure to give an oily residue. This was subjected to medium pressure chromatography on silica using chloroform-methanol gradient elution. The title compound was precipitated with ether to give the free base, m.p. 164—8°C (decomp).

2-[3-(4-Piperidinomethyl)pyrid-2-oxy)propyl]-5-aminopyrimidin-4-one

i) 2-[3-(4-Piperidinomethyl)pyrid-2-oxy)propyl]-5-nitropyrimidin-4-one

To a solution of sodium (0.60 g) in methanol (10 ml) was added a solution of 4-[4-(piperidinomethyl)pyrid-2-oxy]butyronamidine hydrochloride (EP—A—87,274). The mixture was stirred under reflux for 30 minutes, ethyl ethoxymethylenenitroacetate (1.93 g) in methanol (10 ml) was added, and the mixture was stirred under reflux for a further 4 hours. The solvent was evaporated under reduced pressure and water (40 ml) was added to the residue. The mixture was washed with diethyl ether (3 × 20 ml), taken to pH 9 with glacial acetic acid and extracted into chloroform (4 × 25 ml). The combined chloroform extracts were dried ($MgSO_4$) and evaporated under reduced pressure to give an oily residue. This was crystallised from ethanol-ether to give 2-[3-(4-piperidinomethyl)pyrid-2-oxy)propyl]-5-nitropyrimidin-4-one as a pale yellow solid (1.7 g), m.p. 129°—132°C.

ii) 2-[3-(4-(Piperidinomethyl)pyrid-2-oxy)propyl]-5-aminopyrimidin-4-one

To a suspension of 2-[3-(4-piperidinomethyl)pyrid-2-oxy)propyl]-5-nitropyrimidin-4-one (1.43 g) in ethyl acetate (40 ml) was added, in proportions, stannous chloride dihydrate (4.32 g). The mixture was stirred at room temperature for 90 minutes, taken to pH 9 with saturated aqueous sodium bicarbonate and stirred for a further 2 hours. The solid was filtered off and washed with chloroform. The ethyl acetate layer of the filtrate was collected. The aqueous layer of the filtrate was extracted with chloroform (4 × 25 ml). The ethyl acetate and chloroform washings and extracts were combined, dried ($MgSO_4$), and evaporated under reduced pressure to give a residue. This was washed with ether and crystallised from isopropanol-ether to yield 2-[3-(4-(piperidinomethyl)pyrid-2-oxy)propyl]-5-aminopyrimidin-4-one (0.31 g), m.p. 156°—9°C.

Example 8

2-[3-[3-(Hexahydroazepinomethyl)phenoxy]propylamino]-5-aminopyrimidin-4-one

i) 2-[3-[3-(Hexahydroazepinomethyl)phenoxy]propylamino]-5-nitropyrimidin-4-one

3-[3-(Hexahydroazepinomethyl)phenoxy]propylamine (7.20 g) and 2-methylthio-5-nitropyrimidin-4-one (2.57 g) were stirred under reflux in pyridine (40 ml) for 4 hours. The solvent was evaporated under reduced pressure to give a residue that was washed with diethyl ether to afford a yellow solid. Crystallisation from methanol gave 2-[3-[3-(hexahydroazepinomethyl)phenoxy]propylamino]-5-nitropyrimidin-4-one (4.28 g), m.p. 116°—118.5°C.

ii) 2-[3-[3-(Hexahydroazepinomethyl)phenoxy]propylamino]-5-aminopyrimidin-4-one

2-[3-[3-(Hexahydroazepinomethyl)phenoxy]propylamino]-5-nitropyrimidin-4-one (1.00 g) and stannous chloride dihydrate (2.82 g) were stirred in ethanol (25 ml), at room temperature, under nitrogen, for 18 hours. Ethanol was evaporated under reduced pressure, water was added and the mixture taken to pH 8 with aqueous sodium bicarbonate. The mixture was filtered through diatomaceous earth and the insoluble solid washed with chloroform (4 × 50 ml). The combined chloroform extracts were dried ($MgSO_4$) and evaporated under reduced pressure to give the title compound as an oily residue. This was treated with dilute ethanolic HCl and excess solvent evaporated. The residue was crystallised from isopropanol-ethanol to give the title compound as the di-hydrochloride salt (0.29 g) m.p. 169.5°—173.5°C (on recrystallisation from methanol-ethanol).

Example 9

2-[3-(3-Piperidinomethyl)phenoxy)propyl]-5-aminopyrimidin-4-one

To a stirred suspension of 2-[3-(3-piperidinomethyl)-phenoxy)propyl]-5-nitropyrimidin-4-one (3.0 g) in ethyl acetate (200 ml) was added anhydrous stannous chloride (7.8 g). A fawn coloured precipitate resulted. The reaction mixture was stirred at room temperature for one hour after which time a clear solution was observed with an oily precipitate. Saturated aqueous potassium bicarbonate (100 ml) was added and the mixture stirred vigorously for a further 40 minutes giving an emulsion. This was filtered to give two clear layers (aqueous and organic). The aqueous layer was extracted with ethyl acetate. The organic layer and ethyl acetate extractions were combined, washed with brine, dried ($MgSO_4$) and evaporated under reduced pressure to give the title compound as a pale yellow solid (1.75 g).

This was dissolved in ethanol and ethanolic HCl was added. Excess solvent was evaporated and the residue crystallised from methanol to give 2-[3-(3-piperidinomethyl)phenoxy)propyl]-5-aminopyrimidin-4-one dihydrochloride (1.32 g) consistent with the material of Example 2.

In a similar manner the above reaction was conducted in ethanol, on a smaller scale, to give the title product in good yield.

Example 10

2-[3-(3-Piperidinomethyl)phenoxy)propyl]-5-aminopyrimidin-4-one

2-[3-(3-Piperidinomethyl)phenoxy)propyl]-5-nitropyrimidin-4-one (0.5 g) was dissolved in a mixture of water/ammonia (0.88 g) (10.5 ml: 4.5 ml) to give a yellow solution having pH 11. To this stirred solution was added sodium dithionite (1.0 g) and the reaction was stirred for 2 hours at room temperature, further sodium dithionite (0.5 g) was added, the reaction stirred for a further 2 hours and then allowed to stand overnight. The reaction mixture was filtered and the mother liquor left to stand. This afforded a second

precipitate which was collected, dried (0.06 g) and was the title compound, identical by t.l.c. with an authentic sample.

Example 11

2-[3-(3-Piperidinomethyl)phenoxy)propyl]-5-aminopyrimidin-4-one

2-[3-(3-Piperidinomethyl)phenoxy)propyl]-5-nitropyrimidin-4-one (0.1 g) and hydrazine hydrate (0.052 g) were reacted in ethanol (5 ml) in the presence of a small amount of 10% Palladium on carbon, after stirring overnight and heating under reflux for 2 hours, chromatography indicated the presence of the title compound together with impurities and starting-material.

In a similar manner the 5-nitro compound (0.1 g) and hydrazine hydrate (0.06 g) were reacted in ethanol (5 ml) in the presence of a small amount of Raney nickel with similar results.

Example 12

A pharmaceutical composition for oral administration is prepared containing:

| | | % by weight |
|---|---|---|
| A | 2-[3-(3-piperidinomethyl)phenoxy)-propyl]-5-aminopyrimidin-4-one dihydrochloride | 55 |
| A | Dibasic calcium phosphate dihydrate | 20 |
| A | Approved colouring agent | 0.5 |
| | Polyvinylpyrrolidone | 4.0 |
| B | Microcrystalline Cellulose | 8.0 |
| B | Maize Starch | 8.0 |
| B | Sodium glycollate | 4.0 |
| B | Magnesium Stearate | 0.5 |

by mixing together the ingredients A (substituting lactose or microcrystalline cellose for dibasic calcium phosphate dihydrate if desired), adding a concentrated solution of polyvinylpyrrolidone and granulating, drying and screening the dried granules; adding the ingredients B to the dried granules and compressing the mixture into tablets containing 100 mg, 150 mg of 200 mg of the free base.

Other compounds of the invention, for example those specifically described in Examples 1, 3, 4, 6, 7 and 8 can be formulated into pharmaceutical compositions by a similar procedure.

The compounds of this invention, where tested, show no overt signs of toxicity at doses which are a pertinent multiple of the therapeutic dose.

**Claims for the Contracting States: BE CH DE FR GB IT LI LU NL SE**

1. A compound of the formula (I):

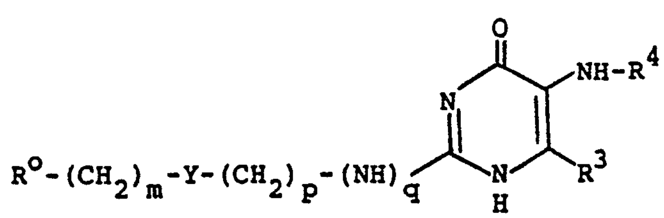

(I)

or a salt thereof, wherein:

$R^o$ is 2-guanidinothiazol-4-yl or a group $R^1R^2N(CH_2)_n$—Z— wherein:

$R^1$ and $R^2$ are independently hydrogen, $C_{1-6}$alkyl, benzyl, phenethyl, furanyl($C_{1-6}$)alkyl, thienyl($C_{1-6}$)alkyl, $C_{3-10}$ cycloalkyl, hydroxy($C_{2-6}$)alkyl, or halo ($C_{2-6}$)alkyl (wherein said hydroxy and halo groups are not substituted on the carbon atom adjacent to the nitrogen atom); or

$R^1$ and $R^2$ together represent —$(CH_2)^r$— wherein r is 4 to 7, to form together with the nitrogen atom to which they are attached a 5—8 membered saturated ring;

n is an integer from 1 to 6;

Z is 2,5-furanyl, 2,5-thienyl, 2,4-pyridyl wherein the $R^1R^2N(CH_2)_n$ group is in the 4-position, 2,4-thiazolyl wherein the $R^1R^2N(CH_2)_n$ group is in the 2-position, or 1,3- or 1,4-phenylene;

m is one; or if Z is pyridyl or phenylene m may also be zero;
Y is oxygen, sulphur or methylene; or if Z is furanyl, thienyl or thiazolyl Y may also be a bond;
p is two, three or four;
q is zero or one;
$R^3$ is hydrogen or $C_{1-6}$alkyl; and
$R^4$ is hydrogen or $C_{1-6}$alkanoyl.

2. A compound according to claim 1 which is a compound of the formula (I) or a pharmaceutically acceptable salt thereof.

3. A compound according to either claim 1 or 2 wherein $R^o$ is a group $R^1R^2N(CH_2)_nZ$— wherein $R^1$, $R^2$, n and Z are as defined in formula (I).

4. A compound according to any one of claims 1 to 3 wherein $R^4$ is hydrogen.

5. A compound according to any one of claims 1 to 4 wherein q is zero.

6. A compound according to any one of claims 1 to 4 wherein q is one.

7. A compound according to any one of claims 1 to 6 wherein $R^3$ is hydrogen.

8. A compound according to any one of claims 1 to 7 wherein $R^1$ and $R^2$ together represent —$(CH_2)^r$— wherein r is 4 to 6.

9. A compound according to any one of claims 1 to 8 wherein $R^1R^2N(CH_2)_n$—Z— is 3-piperidinomethylphenyl-.

10. A compound according to claim 2 which is:
2-[3-(3-(dimethylaminomethyl)phenoxy)propylamino]-5-aminopyrimidin-4-one or a pharmaceutically acceptable salt thereof,
2-[3-(3-(piperidinomethyl)phenoxy)propyl]-5-amino-pyrimidin-4-one or a pharmaceutically acceptable salt thereof,
2-[3-(3-(piperidinomethyl)phenoxy)propylamino]-5-acetamidopyrimidin-4-one or a pharmaceutically acceptable salt thereof,
2-[3-(3-(piperidinomethyl)phenoxy)propylamino]-5-amino-pyrimidin-4-one or a pharmaceutically acceptable salt thereof,
2-[3-(4-(piperidinomethyl)pyrid-2-oxy)propylamino]-5-aminopyrimidin-4-one or a pharmaceutically acceptable salt thereof,
2-[3-(4-(piperidinomethyl)pyrid-2-oxy)propyl]-5-aminopyrimidin-4-one or a pharmaceutically acceptable salt thereof, or
2-[3-[3-(hexahydroazepinomethyl)phenoxy]propylamino]-5-aminopyrimidin-4-one or a pharmaceutically acceptable salt thereof.

11. A compound according to claim 2 which is 2-[3-(3-(piperidinomethyl)phenoxy)propyl]-5-aminopyrimidin-4-one or a pharmaceutically acceptable salt thereof.

12. 2-[3-(3-(Piperidinomethyl)phenoxy)propyl]-5-amino-pyrimidin-4-one dihydrochloride.

13. A pharmaceutical composition which comprises a compound according to any one of claims 2 to 12 and a pharmaceutical acceptable carrier.

14. A compound according to any one of claims 2 to 12 for use as a therapeutic agent.

15. A compound according to any one of claims 2 to 12 for use as an $H_2$-antagonist.

16. A process for preparing a compound of the formula (I) as defined in claim 1 or a salt thereof which process comprises:

a) for compounds of the formula (I) wherein q is one, reacting a compound of the formula (II) with a compound of the formula (III):—

$$R^6(CH_2)_m-Y-(CH_2)_pNH_2$$

(II)

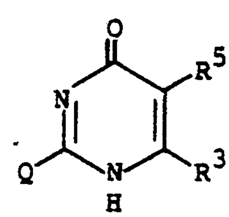

(III)

wherein m, Y, p and $R^3$ are as hereinbefore defined, $R^6$ is a group $R^o$ as hereinbefore defined or $R^6$ is a furan-2-yl or thien-2-yl group; $R^5$ is protected amino; and Q is a group displaceable by amine; or

b) for compounds of the formula (I) wherein m is one and Y is sulphur, reacting a compound of the formula (IV) with a compound of the formula (V):—

$R^6-CH_2-L$

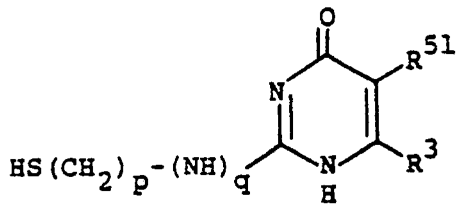

(IV) (V)

wherein $R^6$, p, q and $R^3$ are as hereinbefore defined, $R^{51}$ is optionally protected amino and L is a moiety displaceable by thiol or chemical equivalent thereof; or

c) for compounds of the formula (I) wherein m is one and Y is sulphur, reacting a compound of the formula (VI) or chemical equivalent thereof with a compound of the formula (VII):

$R^6CH_2SH$

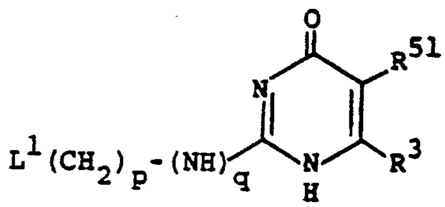

(VI) (VII)

wherein $R^6$, p, q, $R^{51}$ and $R^3$ are as hereinbefore defined and $L^1$ is a moiety displaceable by thiol or chemical equivalent thereof; or

d) reacting a compound of the formula (VIII) with a compound of the formula (IX) or chemical equivalent thereof:

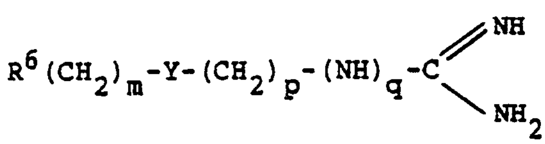

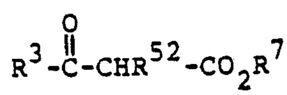

(IX) (VIII)

wherein $R^6$, m, Y, p, q, and $R^3$ are as hereinbefore defined, $R^{52}$ is protected amino and $R^7$ is an ester-forming group; or

e) for compounds of the formula (I) wherein Z is 2,4-pyridyl, m is zero and Y is oxygen, reacting a compound of the formula (X) with a compound of the formula (XI) or derivative thereof that permits reaction to occur:

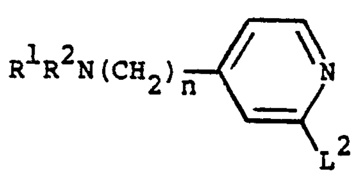

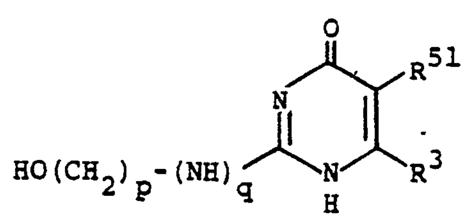

(X) (XI)

wherein $R^1$, $R^2$, n, p, q, $R^3$ and $R^{51}$ are as hereinbefore defined and $L^2$ is a group displaceable by hydroxy or the equivalent thereof; or

f) for compounds of the formula (I) wherein Z is phenylene, m is zero and Y is oxygen, reacting a compound of the formula (XII) or chemical equivalent thereof with a compound of the formula (XIII):

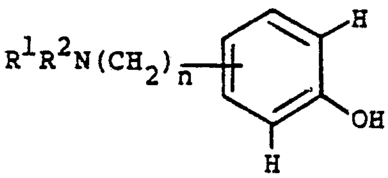

(XII)

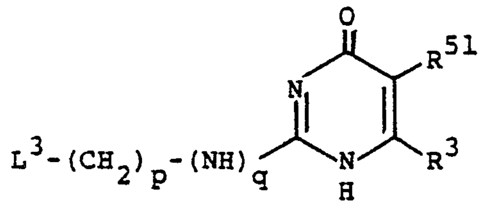

(XIII)

wherein $R^1$, $R^2$, n, p, q, $R^3$ and $R^{51}$ are as hereinbefore defined and $L^3$ is a moiety displaceable by phenol or chemical equivalent thereof; or

g) for compounds of the formula (I) wherein $R^o$ is $R^1R^2N(CH_2)_n$—Z—, converting a compound of the formula (XIV):

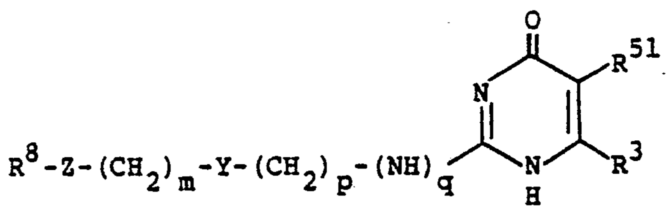

(XIV)

wherein Z, m, Y, p, q, $R^3$ and $R^5$ are as hereinbefore defined and $R^8$ is a precursor of a group $R^1R^2N(CH_2)_n$— as hereinbefore defined; or

h) for compounds of the formula (I) wherein q is one, reducing a compound of the formula (XV):

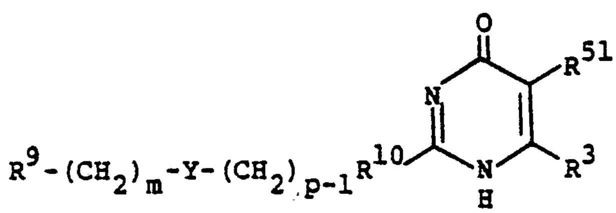

(XV)

wherein m, Y, p, $R^3$ and $R^{51}$ are as hereinbefore defined, $R^9$ is a group $R^o$ or $R^8$—Z— as hereinbefore defined and $R^{10}$ is a group —CH=N— or —CO—NH—;

and thereafter where necessary:

i) reacting a compound wherein $R^6$ is furan-2-yl or thien-2-yl with a Mannich reagent to form a compound of the formula (I) wherein n is one;

ii) converting a protected amino group to amino or $C_{1-6}$alkanoylamino;

iii) optionally forming a salt.

17. A process for preparing a compound of the formula (I) as defined in claim 1 or a salt thereof wherein $R^4$ is hydrogen which comprises reducing a compound of the formula (XVI) or salt thereof:

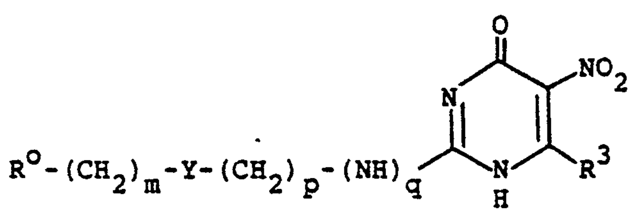

(XVI)

wherein $R^o$, m, Y, p, q and $R^3$ are as defined in claim 1.

18. A process for preparing 2-[3-(3-[piperidinomethyl)phenoxy)propyl]-5-aminopyrimidin-4-one which comprises reducing 2-[3-(3-(piperidinomethyl)phenoxy)propyl]-5-nitropyrimidin-4-one.

19. A process according to either claim 17 or 18 wherein the reducing agent comprises stannous chloride.

20. A process according to claim 19 wherein the solvent comprises ethyl acetate and stannous chloride is in anhydrous form.

21. A compound of the formula (XVI):

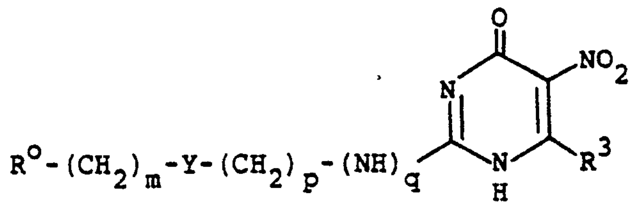

(XVI)

or a salt thereof, wherein R°, m, Y, p, q and $R^3$ are as defined in claim 1.

22. 2-[3-(3-Piperidinomethyl)phenoxy)propyl]-5-nitropyrimidin-4-one.

**Claims for the Contracting State: AT**

1. A process for preparing a compound of the formula (I):—

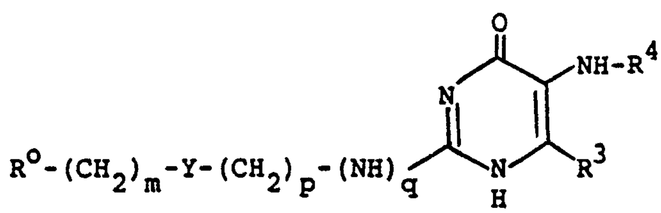

(I)

or a salt thereof, wherein:

R° is 2-guanidinothiazol-4-yl or a group $R^1R^2N(CH_2)_n$—Z— wherein:

$R^1$ and $R^2$ are independently hydrogen, $C_{1-6}$alkyl, benzyl, phenethyl, furanyl($C_{1-6}$)alkyl, thienyl($C_{1-6}$)alkyl, $C_{3-10}$ cycloalkyl, hydroxy($C_{2-6}$)alkyl, or halo ($C_{2-6}$)alkyl (wherein said hydroxy and halo groups are not substituted on the carbon atom adjacent to the nitrogen atom); or

$R^1$ and $R^2$ together represent —$(CH_2)^r$— wherein r is 4 to 7, to form together with the nitrogen atom to which they are attached a 5—8 membered saturated ring;

n is an integer from 1 to 6;

Z is 2,5-furanyl, 2,5-thienyl, 2,4-pyridyl wherein the $R^1R^2N(CH_2)_n$ group is in the 4-position, 2,4-thiazolyl wherein the $R^1R^2N(CH_2)_n$ group is in the 2-position, or 1,3- or 1,4-phenylene;

m is one; or if Z is pyridyl or phenylene m may also be zero;

Y is oxygen, sulphur or methylene; or if Z is furanyl, thienyl or thiazolyl Y may also be a bond;

p is two, three or four;

q is zero or one;

$R^3$ is hydrogen or $C_{1-6}$alkyl; and

$R^4$ is hydrogen or $C_{1-6}$alkanoyl.

which process comprises:

a) for compounds of the formula (I) wherein q is one, reacting a compound of the formula (II) with a compound of the formula (III):—

$$R^6(CH_2)_m\text{-}Y\text{-}(CH_2)_pNH_2$$

(II)

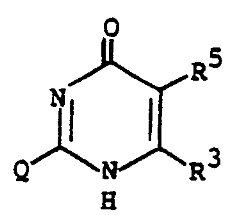

(III)

wherein m, Y, p and $R^3$ are as hereinbefore defined, $R^6$ is a group R° as hereinbefore defined or $R^6$ is a furan-2-yl or thien-2-yl group; $R^5$ is protected amino; and Q is a group displaceable by amine; or

b) for compounds of the formula (I) wherein m is one and Y is sulphur, reacting a compound of the formula (IV) with a compound of the formula (V):—

$R^6-CH_2-L$

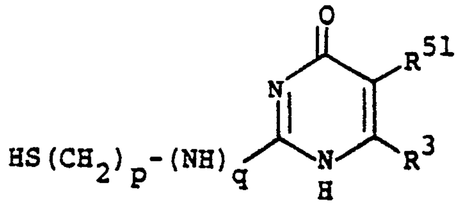

(IV) (V)

wherein $R^6$, p, q and $R^3$ are as hereinbefore defined, $R^{51}$ is optionally protected amino and L is a moiety displaceable by thiol or chemical equivalent thereof; or

c) for compounds of the formula (I) wherein m is one and Y is sulphur, reacting a compound of the formula (VI) or chemical equivalent thereof with a compound of the formula (VII):

$R^6CH_2SH$

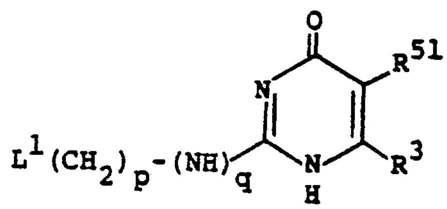

(VI) (VII)

wherein $R^6$, p, q, $R^{51}$ and $R^3$ are as hereinbefore defined and $L^1$ is a moiety displaceable by thiol or chemical equivalent thereof; or

d) reacting a compound of the formula (VIII) with a compound of the formula (IX) or chemical equivalent thereof:

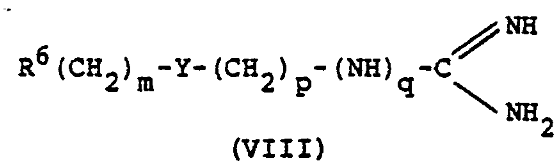

(VIII)

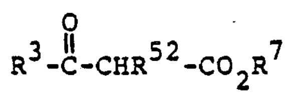

(IX)

wherein $R^6$, m, Y, p, q, and $R^3$ are as hereinbefore defined, $R^{52}$ is protected amino and $R^7$ is an ester-forming group; or

e) for compounds of the formula (I) wherein Z is 2,4-pyridyl, m is zero and Y is oxygen, reacting a compound of the formula (X) with a compound of the formula (XI) or derivative thereof that permits reaction to occur:

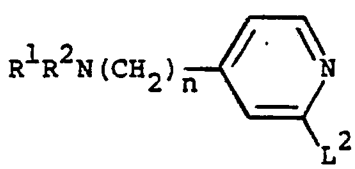

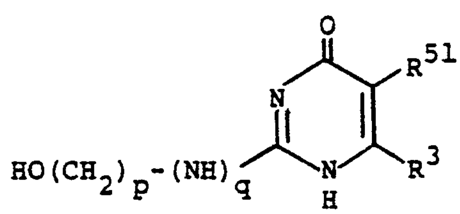

(X) (XI)

wherein $R^1$, $R^2$, n, p, q, $R^3$ and $R^{51}$ are as hereinbefore defined and $L^2$ is a group displaceable by hydroxy or the equivalent thereof; or

f) for compounds of the formula (I) wherein Z is phenylene, m is zero and Y is oxygen, reacting a compound of the formula (XII) or chemical equivalent thereof with a compound of the formula (XIII):

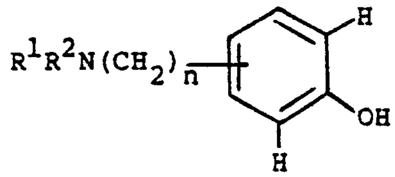

(XII)

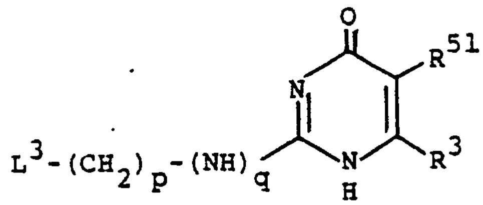

(XIII)

wherein $R^1$, $R^2$, n, p, q, $R^3$ and $R^{51}$ are as hereinbefore defined and $L^3$ is a moiety displaceable by phenol or chemical equivalent thereof; or

g) for compounds of the formula (I) wherein $R^0$ is $R^1R^2N(CH_2)_n$—Z—, converting a compound of the formula (XIV):

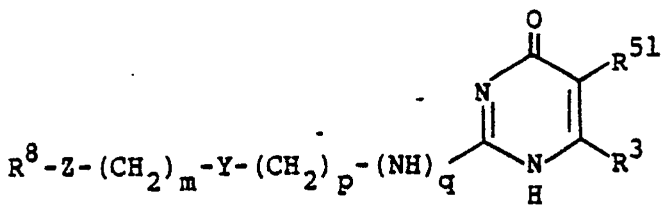

(XIV)

wherein Z, m, Y, p, q, $R^3$ and $R^5$ are as hereinbefore defined and $R^8$ is a precursor of a group $R^1R^2N(CH_2)_n$— as hereinbefore defined; or

h) for compounds of the formula (I) wherein q is one, reducing a compound of the formula (XV):

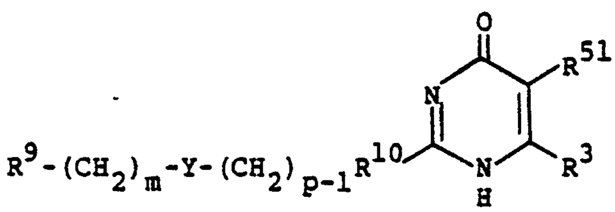

(XV)

wherein m, Y, p, $R^3$ and $R^{51}$ are as hereinbefore defined, $R^9$ is a group $R^0$ or $R^8$—Z— as hereinbefore defined and $R^{10}$ is a group —CH=N— or —CO—NH—;

and thereafter where necessary:

i) reacting a compound wherein $R^6$ is furan-2-yl or thien-2-yl with a Mannich reagent to form a compound of the formula (I) wherein n is one;

ii) converting a protected amino group to amino or $C_{1-6}$alkanoylamino;

iii) optionally forming a salt.

2. A process according to claim 1 for preparing a compound of the formula (I) as salt thereof wherein $R^0$ is a group $R^1R^2N(CH_2)_nZ$— wherein $R^1$, $R^2$, n and Z are as defined in formula (I) when performed according to any of variants a)—d).

3. A process according to either claim 1 or 2 for preparing a compound of the formula (I) or salt thereof wherein $R^4$ is hydrogen.

4. A process according to any one of claims 1 to 3 for preparing a compound of the formula (I) or salt thereof wherein q is zero.

5. A process according to any one of claims 1 to 3 for preparing a compound of the formula (I) or salt thereof wherein q is one.

6. A process according to any one of claims 1 to 5 for preparing a compound of the formula (I) or salt thereof wherein $R^3$ is hydrogen.

7. A process according to any one of claims 1 to 6 for preparing a compound of the formula (I) or salt thereof wherein $R^1$ and $R^2$ together represent $—(CH_2)^r$ wherein r is 4 to 6.

8. A process according to any one of claims 1 to 7 for preparing a compound of the formula (I) or salt thereof wherein $R^1R^2N(CH_2)_n$—Z— is 3-piperidinomethylphenyl-.

9. A process according to claim 1 for preparing:

2-[3-(3-(dimethylaminomethyl)phenoxy)propylamino]-5-aminopyrimidin-4-one or a pharmaceutically acceptable salt thereof,

2-[3-(3-(piperidinomethyl)phenoxy)propyl]-5-amino-pyrimidin-4-one or a pharmaceutically acceptable salt thereof,

2-[3-(3-(piperidinomethyl)phenoxy)propylamino]-5-acetamidopyrimidin-4-one or a pharmaceutically acceptable salt thereof,

2-[3-(3-(piperidinomethyl)phenoxy)propylamino]-5-amino-pyrimidin-4-one or a pharmaceutically acceptable salt thereof,

2-[3-(4-(piperidinomethyl)pyrid-2-oxy)propylamino]-5-aminopyrimidin-4-one or a pharmaceutically acceptable salt thereof,

2-[3-(4-(piperidinomethyl)pyrid-2-oxy)propyl]-5-aminopyrimidin-4-one or a pharmaceutically acceptable salt thereof, or

2-[3-[3-(hexahydroazepinomethyl)phenoxy]propylamino]-5-aminopyrimidin-4-one or a pharmaceutically acceptable salt thereof.

10. A process according to claim 1 for preparing 2-[3-(3-(piperidinomethyl)phenoxy)propyl]-5-amino-pyrimidin-4-one or a pharmaceutically acceptable salt thereof.

11. A process for preparing a compound of the formula (I) as defined in claim 1 or a salt thereof wherein $R^4$ is hydrogen which comprises reducing a compound of the formula (XVI) or salt thereof:

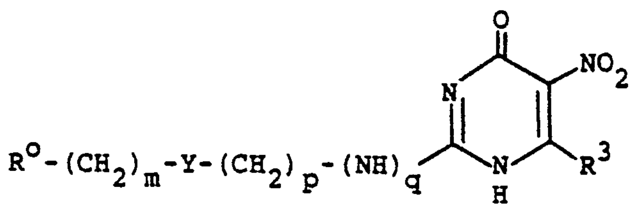

(XVI)

wherein R°, m, Y, p, q and $R^3$ are as defined in claim 1.

12. A process for preparing 2-[3-(3-(piperidinomethyl)phenoxy)propyl]-5-aminopyrimidin-4-one which comprises reducing 2-[3-(3-(piperidinomethyl)phenoxy)propyl]-5-nitropyrimidin-4-one.

13. A process according to either claim 11 or 12 wherein the reducing agent comprises stannous chloride under substantially neutral conditions.

14. A process according to claim 13 wherein the solvent comprises ethyl acetate and stannous chloride is in anhydrous form.

15. A process for preparing a pharmaceutical composition which comprises bringing into association a compound of the formula (I) as defined in claim 1 or a pharmaceutically acceptable salt thereof and a pharmaceutically acceptable carrier.

16. A process according to claim 15 wherein the compound of the formula (I) is 2-[3-(3-piperidinomethyl)phenoxy)propyl]-5-aminopyrimidin-4-one.

**Patentansprüche für die Vertragsstaaten: BE CH DE FR GB IT LI LU NL SE**

1. Verbindung der Formel I

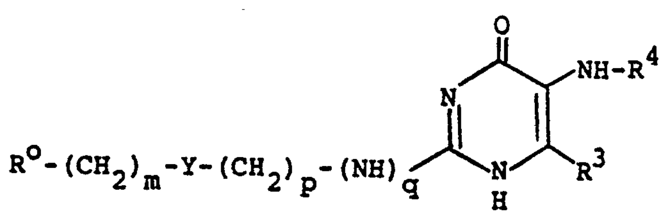

(I)

oder ein Salz davon, in der:

R° eine 2-Guanidinothiazol-4-ylgruppe oder eine Gruppe $R^1R^2N(CH_2)_n$—Z— bedeutet, in der:

$R^1$ und $R^2$ unabhängig voneinander ein Wasserstoffatom, einen $C_{1-6}$-Alkylrest, eine Benzyl-, Phenäthylgruppe, einen Furanyl ($C_{1-6}$)-alkyl-, Thienyl ($C_{1-6}$)-alkyl-, $C_{3-10}$-Cycloalkyl-, Hydroxy ($C_2$—$_6$)-alkyl- oder einen Halogen ($C_{2-6}$)-alkylrest bedeuten (wobe die Hydroxyl- und Halogengruppen nicht am zur Stickstoffatom benachbarten Kohlenstoffatom substituiert sind); oder

$R^1$ und $R^2$ zusammen eine Gruppe —$(CH_2)^r$— bedeuten, in der r einen Wert von 4 bis 7 hat, so daß sie zusammen mit dem Stickstoffatom, an das sie gebunden sind, einen 5- bis 8-gliedrigen, gesättigten Ring bilden;

n eine ganze Zahl mit einem Wert von 1 bis 6 ist;

Z eine 2,5-Furanyl-, 2,5-Thienylgruppe, eine 2,4-Pyridylgruppe, in der sich die Gruppe $R^1R^2N(CH_2)_n$ in der 4-Stellung befindet, eine 2,4-Thiazolylgruppe, in der sich die Gruppe $R^1R^2N(CH_2)_n$ in der 2-Stellung befindet oder eine 1,3- oder 1,4-Phenylengruppe bedeutet;

m den Wert 1 hat; oder, falls Z eine Pyridyl- oder Phenylengruppe ist, m auch den Wert Null haben kann;

Y ein Sauerstoff- oder Schwefelatom oder eine Methylengruppe bedeutet; oder, falls Z eine Furanyl-, Thienyl- oder Thiazolylgruppe ist, Y auch eine Bindung bedeuten kann;

p den Wert 2, 3 oder 4 hat;
q den Wert 0 oder 1 hat;
$R^3$ ein Wasserstoffatom oder ein $C_{1-6}$-Alkylrest ist; und
$R^4$ ein Wasserstoffatom oder ein $C_{1-6}$-Alkanoylrest ist.

2. Verbindung nach Anspruch 1, die eine Verbindung der Formel I oder ein pharmazeutisch verträgliches Salz davon ist.

3. Verbindung nach Anspruch 1 oder 2, in der $R^0$ eine Gruppe $R^1R^2N(CH_2)_nZ—$ ist, in der $R^1$, $R^2$, n und Z die bei Formel I angegebene Bedeutung haben.

4. Verbindung nach einem der Ansprüche 1 bis 3, in der $R^4$ ein Wasserstoffatom ist.

5. Verbindung nach einem der Ansprüche 1 bis 4, in der q den Wert Null hat.

6. Verbindung nach einem der Ansprüche 1 bis 4, in der q den Wert 1 hat.

7. Verbindung nach einem der Ansprüche 1 bis 6, in der $R^3$ ein Wasserstoffatom ist.

8. Verbindung nach einem der Ansprüche 1 bis 7, in der $R^1$ und $R^2$ zusammen eine Gruppe $—(CH_2)^r—$ bedeuten, in der r einen Wert von 4 bis 6 hat.

9. Verbindung nach einem der Ansprüche 1 bis 8, in der $R^1R^2N(CH_2)_n—Z—$ eine 3-Piperidinomethylphenylgruppe ist.

10. Verbindung nach Anspruch 2, dadurch gekennzeichnet, daß sie
2-[3-(3-(Dimethylaminomethyl)phenoxy)propylamino]-5-aminopyrimidin-4-on oder ein pharmazeutisch verträgliches Salz davon,
2-[3-(3-(Piperidinomethyl)phenoxy)propyl]-5-aminopyrimidin-4-on oder ein pharmazeutisch verträgliches Salz davon,
2-[3-(3-Piperidinomethyl)phenoxy)propylamino]-5-acetamidopyrimidin-4-on oder ein pharmazeutisch vertragliches Salz davon,
2-[3-(3-Piperidinomethyl)phenoxy)propylamino]-5-aminopyrimidin-4-on oder ein pharmazeutisch verträgliches Salz davon,
2-[3-(4-Piperidinomethyl)pyrid-2-oxy)propylamino]-5-aminopyrimidin-4-on oder ein pharmazeutisch verträgliches Salz davon,
2-[3-(4-Piperidinomethyl)pyrid-2-oxy)propyl]-5-aminopyrimidin-4-on oder ein pharmazeutisch verträgliches Salz davon, oder
2-[3-[3-(Hexahydrazepinomethyl)phenoxy]propylamino]-5-aminopyrimidin-4-on oder ein pharmazeutisch verträgliches Salz davon ist.

11. Verbindung nach Anspruch 2, dadurch gekennezeichnet, daß 2-[3-(3-(Piperidinomethyl)phenoxy)-propyl]-5-aminopyrimidin-4-on oder ein pharmazeutisch verträgliches Salz davon ist.

12. 2-[3-(3-[Piperidinomethyl)phenoxy)propyl]-5-aminopyrimidin-4-on-dihydrochlorid.

13. Arzneimittel enthaltend eine Verbindung nach einem der Ansprüche 2 bis 12 und einen pharmazeutisch verträglichen Träger.

14. Verbindung nach einem der Ansprüche 2 bis 12 zur Verwendung als Therapeutikum.

15. Verbindung nach einem der Ansprüche 2 bis 12 zur Verwendung als $H_2$-Antagonist.

16. Verfahren zur Herstellung einer Verbindung der im Anspruch 1 angegebenen Formel I oder eines Salzes davon, bei dem man:

a) zur Herstellung von Verbindungen der Formel I, in der q den Wert 1 hat, eine Verbindung der Formel II

$$R^6(CH_2)_m—Y—(CH_2)_pNH_2 \qquad (II)$$

mit einer Verbindung der Formel III

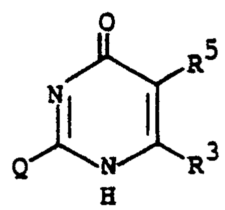

(III)

umsetzt, wobei m, Y, p und $R^3$ die vorstehend angegebene Bedeutung haben, $R^6$ ein wie vorstehend angegebener Rest $R^0$, oder $R^6$ eine Furan-2-yl- oder Thien-2-ylgruppe ist; $R^5$ eine mit einer Schutzgruppe versehene Aminogruppe bedeutet; und Q eine durch eine Aminogruppe ersetzbare Gruppe ist; oder

b) zur Herstellung von Verbindungen der allgemeinen Formel I, in der m den Wert 1 hat und Y ein Schwefelatom ist, eine Verbindung der Formel IV

$$R^6—CH_2—L \qquad (IV)$$

mit einer Verbindung der Formel V

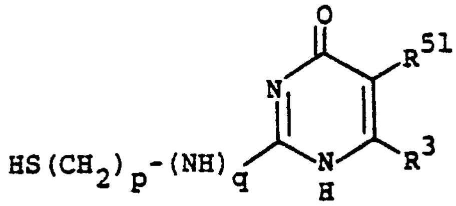

(V)

umsetzt, wobei $R^6$, p, q und $R^3$ die vorstehend angegebene Bedeutung haben, $R^{51}$ eine gegebenenfalls mit einer Schutzgruppe versehene Aminogruppe ist und L ein durch eine Thiolgruppe oder ein chemisches Äquivalent davon ersetzbarer Rest ist; oder

c) zur Herstellung von Verbindungen der Formel I, in der m den Wert 1 hat und Y ein Schwefelatom ist, eine Verbindung der Formel VI

$$R^6CH_2SH \qquad (VI)$$

oder ein chemisches Äquivalent davon mit einer Verbindung der Formel VII

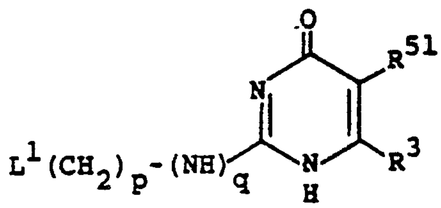

(VII)

umsetzt, wobei $R^6$, p, q, $R^{51}$ und $R^3$ die vorstehend angegebene Bedeutung haben und $L^1$ ein durch eine Thiolgruppe oder ein chemisches Äquivalent davin ersetzbarer Rest ist; oder

d) eine Verbindung der Formel VIII

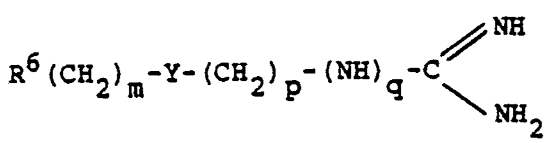

(VIII)

mit einer Verbindung der Formel IX

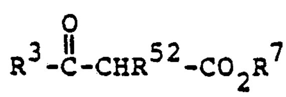

(IX)

oder mit einem chemischen Äquivalent davon umsetzt, wobei $R^6$, m, Y, p, q und $R^3$ die vorstehend angegebene Bedeutung haben, $R^{52}$ eine mit einer Schutzgruppe versehene Aminogruppe ist und $R^7$ eine Ester-bildende Gruppe bedeutet; oder

e) zur Herstellung von Verbindungen der Formel I, in der Z eine 2,4-Pyridylgruppe ist, m den wert Null hat und Y ein Sauerstoffatom bedeutet, eine Verbindung der Formel X

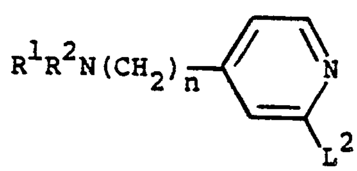

(X)

mit einer Verbindung der Formel XI

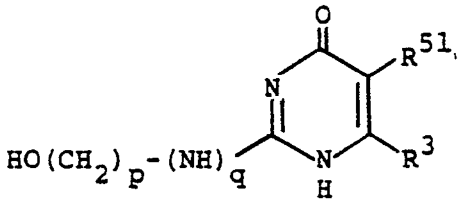

(XI)

oder mit eimem Derivat davon umsetzt, das diese Umsetzung gestattet, wobei $R^1$, $R^2$, n, p, q, $R^3$ und $R^{51}$ die vorstehend angegebene Bedeutung haben und $L^2$ eine durch eine Hydroxylgruppe oder ein Äquivalent davon ersetzbare Gruppe ist; oder

f) zur Herstellung von Verbindungen der Formel I, in der Z eine Phenylengruppe ist, m den Wert Null hat und Y ein Sauerstoffatom bedeutet, eine Verbindung der Formel XII

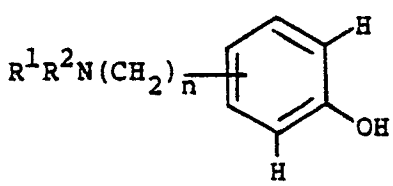

(XII)

oder ein chemisches Äquivalent davon mit einer Verbindung der Formel XIII

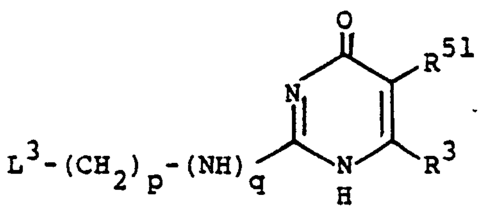

(XIII)

umsetzt, wobei $R^1$, $R^2$, n, p, q, $R^3$ und $R^{51}$ die vorstehend angegebene Bedeutung haben und $L^3$ ein durch eine Phenolgruppe oder ein chemisches Äquivalent davon ersetzbarer Rest ist; oder

g) zur Herstellung von Verbindungen der Formel I, in der $R^o$ eine Gruppe $R^1R^2N(CH_2)_n$—Z— ist, eine Verbindung der Formel XIV

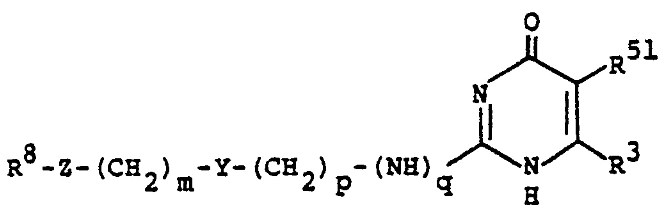

(XIV)

unsetzt, in der Z, m, Y, p, q, $R^3$ und $R^5$ die vorstehend angegebene Bedeutung haben, und $R^8$ eine Vorstufe einer wie vorstehend angegebenen Gruppe $R^1R^2N(CH_2)_n$— ist; oder

h) zur Herstellung von Verbindungen der Formel I, in der q den Wert 1 hat, eine Verbindung der Formel XV

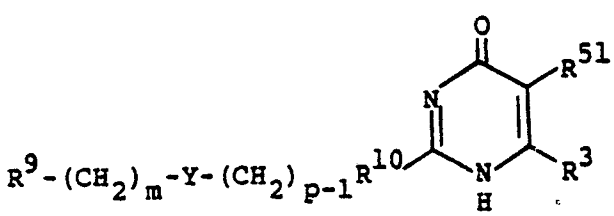

(XV)

reduziert, in der m, Y, p, $R^3$ und $R^{51}$ die vorstehend angegebene Bedeutung haben, $R^9$ eine wie vorstehend angegebene Gruppe $R^o$ oder eine wie vorstehend angegebene Gruppe $R^8$—Z— ist und $R^{10}$ eine Gruppe —CH=N— oder —CO—NH— ist;

um sodann gegebenenfalls:

i) eine Verbindung, in der $R^6$ eine Furan-2-yl- oder eine Thien-2-ylgruppe ist, mit einem Mannich-Reagens umsetzt, um eine Verbindung der Formel I herzustellen, in der n den Wert 1 hat;

ii) eine mit einer Schutzgruppe versehen Aminogruppe in die Aminogruppe oder in einen $C_{1-6}$-Alkanoylaminorest überführt;

iii) gegebenenfalls ein Salz bildet.

17. Verfahren zur Herstellung einer Verbindung der in Anspruch 1 angegebenen Bedeutung der Formel I oder eines Salzes davon, in der $R^4$ ein Wasserstoffatom ist, bei dem man eine Verbindung der Formel XVI

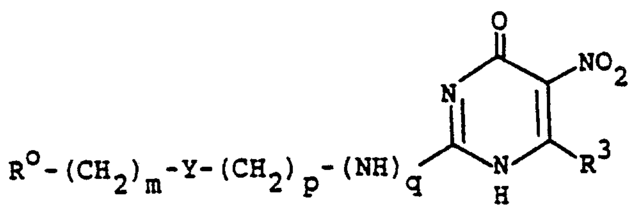

(XVI)

in der R°, m, Y, p, q und $R^3$ die in Anspruch 1 angegebene Bedeutung haben, oder ein Salz davon reduziert.

18. Verfahren zur Herstellung von 2-[3-(3-(Piperidinomethyl)phenoxy)propyl]-5-aminopyrimidin-4-on, bei dem man 2-[3-(3-(Piperidinomethyl)phenoxy)propyl]-5-nitropyrimidin-4-on reduziert.

19. Verbindung nach Anspruch 17 oder 18, bei dem das Reduktionsmittel Zinn(II)-chlorid enthält.

20. Verfahren nach Anspruch 19, bei dem das Lösungsmittel Äthylacetat enthält und das Zinn(II)-chlorid in wasserfreier Form vorliegt.

21. Verbindung der Formel XVI

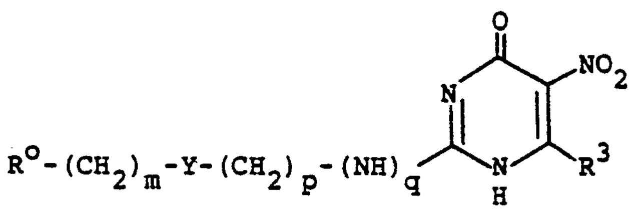

(XVI)

oder ein Salz davon, in der R°, m, Y, p, q und $R^3$ die in Anspruch 1 angegebene Bedeutung haben.

22. 2-[3-(3-(Piperidinomethyl)phenoxy)propyl]-5-nitropyrimidin-4-on.

**Patentansprüche für den Vertragsstaat: AT**

1. Verfahren zur Herstellung einer Verbindung der Formel I

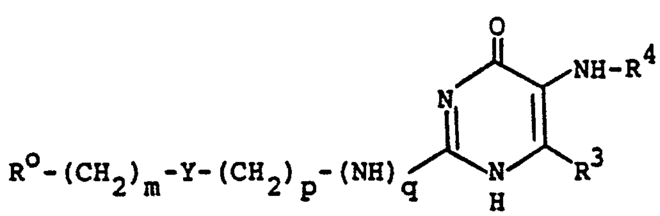

(I)

oder eines Salzes davon, in der:

R° eine 2-Guanidinothiazol-4-ylgruppe oder eine Gruppe $R^1R^2N(CH_2)_n$—Z— bedeutet, in der:

$R^1$ und $R^2$ unabhängig voneinander ein Wasserstoffatom, einen $C_{1-6}$-Alkylrest, eine Benzyl-, Phenäthylgruppe, einen Furanyl $(C_{1-6})$-alkyl-, Thienyl $(C_{1-6})$-alkyl-, $C_{3-10}$-Cycloalkyl-, Hydroxy $(C_2—_6)$-alkyl- oder einen Halogen $(C_{2-6})$-alkylrest bedeuten (wobe die Hydroxyl- und Halogengruppen nicht am zur Stickstoffatom benachbarten Kohlenstoffatom substituiert sind); oder

$R^1$ und $R^2$ zusammen eine Gruppe $—(CH_2)^r—$ bedeuten, in der r einen Wert von 4 bis 7 hat, so daß sie zusammen mit dem Stickstoffatom, an das sie gebunden sind, einen 5- bis 8-gliedrigen, gesättigten Ring bilden;

n eine ganze Zahl mit einem Wert von 1 bis 6 ist;

Z eine 2,5-Furanyl-, 2,5-Thienylgruppe, eine 2,4-Pyridylgruppe, in der sich die Gruppe $R^1R^2N(CH_2)_n$ in der 4-Stellung befindet, eine 2,4-Thiazolylgruppe, in der sich die Gruppe $R^1R^2N(CH_2)_n$ in der 2-Stellung befindet oder eine 1,3- oder 1,4-Phenylengruppe bedeutet;

m den Wert 1 hat; oder, falls Z eine Pyridyl- oder Phenylengruppe ist, m auch den Wert Null haben kann;

Y ein Sauerstoff- oder Schwefelatom oder eine Methylengruppe bedeutet; oder, falls Z eine Furanyl-, Thienyl- oder Thiazolylgruppe ist, Y auch eine Bindung bedeuten kann;

p den Wert 2, 3 oder 4 hat;

q den Wert 0 oder 1 hat;

$R^3$ ein Wasserstoffatom oder ein $C_{1-6}$-Alkylrest ist; und

$R^4$ ein Wasserstoffatom oder ein $C_{1-6}$-Alkanoylrest ist;

bei dem man

a) zur Herstellung von Verbindungen der Formel I, in der q den Wert 1 hat, eine Verbindung der Formel II

$$R^6(CH_2)_m—Y—(CH_2)_pNH_2 \qquad (II)$$

mit einer Verbindung der Formel III

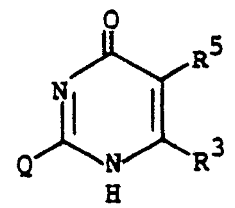

(III)

umsetzt, wobei m, Y, p und $R^3$ die vorstehend angegebene Bedeutung haben, $R^6$ ein wie vorstehend angegebener Rest $R^o$, oder $R^6$ eine Furan-2-yl- oder Thien-2-ylgruppe ist; $R^5$ eine mit einer Schutzgruppe versehene Aminogruppe bedeutet; und Q eine durch eine Aminogruppe ersetzbare Gruppe ist; oder

b) zur Herstellung von Verbindungen der allgemeinen Formel I, in der m den Wert 1 hat und Y ein Schwefelatom ist, eine Verbindung der Formel IV

$$R^6—CH_2—L \qquad (IV)$$

mit einer Verbindung der Formel V

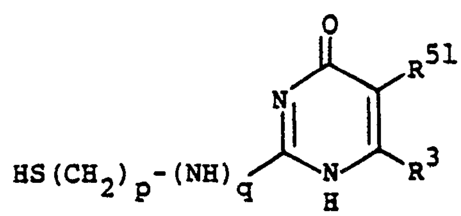

(V)

umsetzt, wobei $R^6$, p, q und $R^3$ die vorstehend angegebene Bedeutung haben, $R^{51}$ eine gegebenenfalls mit einer Schutzgruppe versehene Aminogruppe ist und L ein durch eine Thiolgruppe oder ein chemisches Äquivalent davon ersetzbarer Rest ist; oder

c) zur Herstellung von Verbindungen der Formel I, in der m den Wert 1 hat und Y ein Schwefelatom ist, eine Verbindung der Formel VI

$$R^6CH_2SH \qquad (VI)$$

oder ein chemisches Äquivalent davon mit einer Verbindung der Formel VII

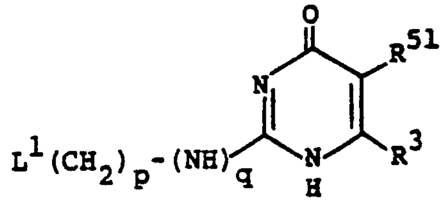

(VII)

umsetzt, wobei $R^6$, p, q, $R^{51}$ und $R^3$ die vorstehend angegebene Bedeutung haben und $L^1$ ein durch eine Thiolgruppe oder ein chemisches Äquivalent davin ersetzbarer Rest ist; oder

d) eine Verbindung der Formel VIII

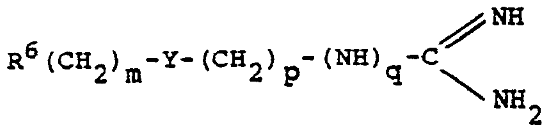

(VIII)

mit einer Verbindung der Formel IX

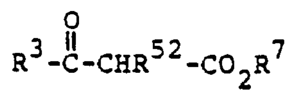

(IX)

oder mit einem chemischen Äquivalent davon umsetzt, wobei $R^6$, m, Y, p, q und $R^3$ die vorstehend angegebene Bedeutung haben, $R^{52}$ eine mit einer Schutzgruppe versehene Aminogruppe ist und $R^7$ eine Ester-bildende Gruppe bedeutet; oder

e) zur Herstellung von Verbindungen der Formel I, in der Z eine 2,4-Pyridylgruppe ist, m den wert Null hat und Y ein Sauerstoffatom bedeutet, eine Verbindung der Formel X

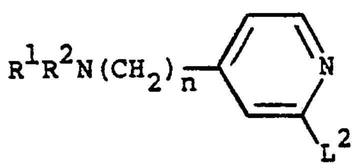

(X)

mit einer Verbindung der Formel XI

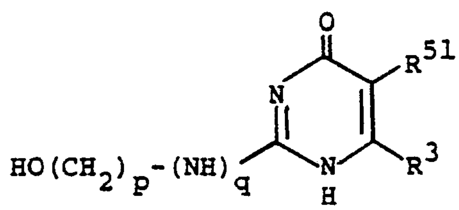

(XI)

oder mit eimem Derivat davon umsetzt, das diese Umsetzung gestattet, wobei $R^1$, $R^2$, n, p, q, $R^3$ und $R^{51}$ die vorstehend angegebene Bedeutung haben und $L^2$ eine durch eine Hydroxylgruppe oder ein Äquivalent davon ersetzbare Gruppe ist; oder

f) zur Herstellung von Verbindungen der Formel I, in der Z eine Phenylengruppe ist, m den Wert Null hat und Y ein Sauerstoffatom bedeutet, eine Verbindung der Formel XII

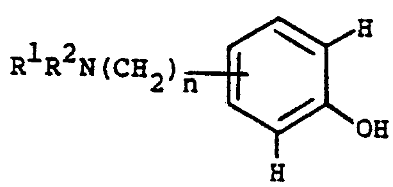

(XII)

oder ein chemisches Äquivalent davon mit einer Verbindung der Formel XIII

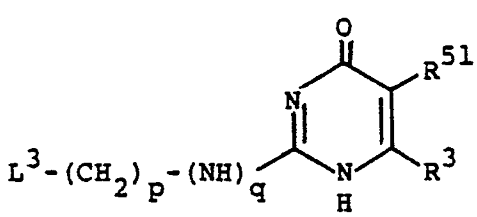

(XIII)

umsetzt, wobei $R^1$, $R^2$, n, p, q, $R^3$ und $R^{51}$ die vorstehend angegebene Bedeutung haben und $L^3$ ein durch eine Phenolgruppe oder ein chemisches Äquivalent davon ersetzbarer Rest ist; oder

g) zur Herstellung von Verbindungen der Formel I, in der $R^o$ eine Gruppe $R^1R^2N(CH_2)_n—Z—$ ist, eine, Verbindung der Formel XIV

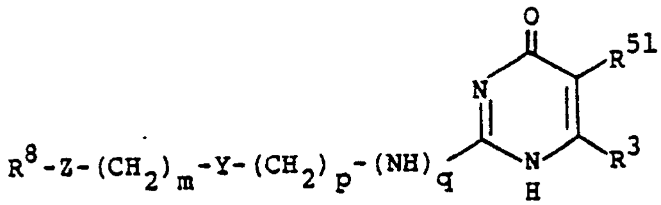

(XIV)

unsetzt, in der Z, m, Y, p, q, $R^3$ und $R^5$ die vorstehend angegebene Bedeutung haben, und $R^8$ eine Vorstufe einer wie vorstehend angegebenen Gruppe $R^1R^2N(CH_2)_n—$ ist; oder

h) zur Herstellung von Verbindungen der Formel I, in der q den Wert 1 hat, eine Verbindung der Formel XV

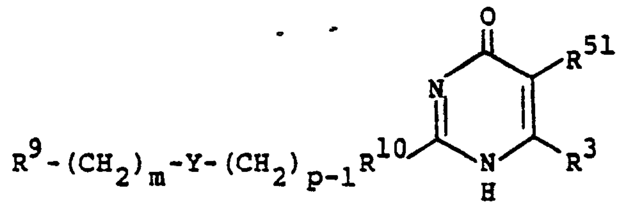

(XV)

reduziert, in der m, Y, p, $R^3$ und $R^{51}$ die vorstehend angegebene Bedeutung haben, $R^9$ eine wie vorstehend angegebene Gruppe $R^o$ oder eine wie vorstehend angegebene Gruppe $R^8—Z—$ ist und $R^{10}$ eine Gruppe —CH=N— oder —CO—NH— ist;

um sodann gegebenenfalls:

i) eine Verbindung, in der $R^6$ eine Furan-2-yl- oder eine Thien-2-ylgruppe ist, mit einem Mannich-Reagens umsetzt, um eine Verbindung der Formel I herzustellen, in der n den Wert 1 hat;

ii) eine mit einer Schutzgruppe versehen Aminogruppe in die Aminogruppe oder in einen $C_{1-6}$-Alkanoylaminorest überführt;

iii) gegebenenfalls ein Salz bildet.

2. Verfahren nach Anspruch 1, zur Herstellung einer Verbindung der Formel I oder eines Salzes davon, in der $R^o$ eine Gruppe $R^1R^2N(CH_2)_n—Z—$ ist, in der $R^1$, $R^2$, n und Z die bei der Formel I angegebene Bedeutung haben, sofern es nach einer der Ausführungsformen a) bis d) durchgeführt wird.

3. Verfahren nach einem der Ansprüche 1 oder 2 zur Herstellung einer Verbindung der Formel I oder eines Salzes davon, in der $R^4$ ein Wasserstoffatom ist.

4. Verfahren nach einem der Ansprüche 1 bis 3 zur Herstellung einer Verbindung der Formel I oder eines Salzes davon, in der q den Wert Null hat.

5. Verfahren nach einem der Ansprüche 1 bis 3 zur Herstellung einer Verbindung der Formel I oder eines Salzes davon, in der q den Wert 1 hat.

6. Verfahren nach einem der Ansprüche 1 bis 5 zur Herstellung einer Verbindung der Formel I oder eines Salzes davon, in der $R^3$ ein Wasserstoffatom ist.

7. Verfahren nach einem der Ansprüche 1 bis 6 zur Herstellung einer Verbindung der Formel I oder eines Salzes davon, in der $R^1$ und $R^2$ zusammen eine Gruppe $—(CH_2)^r—$ bilden, in der r den Wert 4 bis 6 hat.

8. Verfahren nach einem der Ansprüche 1 bis 7 zur Herstellung einer Verbindung der Formel I oder eines Salzes davon, in der die Gruppe $R^1R^2N(CH_2)_n—Z—$ eine 3-Piperidinomethylphenylgruppe ist.

9. Verfahren nach Anspruch 1 zur Herstellung von:

2-[3-(3-(Dimethylaminomethyl)phenoxy)propylamino]-5-aminopyrimidin-4-on oder eines pharmazeutisch verträglichen Salzes davon,

2-[3-(3-(Piperidinomethyl)phenoxy)propyl]-5-aminopyrimidin-4-on oder eines pharmazeutisch verträglichen Salzes davon,

2-[3-(3-Piperidinomethyl)phenoxy)propylamino]-5-acetamidopyrimidin-4-on oder eines pharmazeutisch vertraglichen Salzes davon,

2-[3-(3-Piperidinomethyl)phenoxy)propylamino]-5-aminopyrimidin-4-on oder eines pharmazeutisch verträglichen Salzes davon,

2-[3-(4-Piperidinomethyl)pyrid-2-oxy)propylamino]-5-aminopyrimidin-4-on oder eines pharmazeutisch verträglichen Salzes davon,

2-[3-(4-Piperidinomethyl)pyrid-2-oxy)propyl]-5-aminopyrimidin-4-on oder eines pharmazeutisch verträglichen Salzes davon, oder

2-[3-[3-(Hexahydrazepinomethyl)phenoxy]propylamino]-5-aminopyrimidin-4-on oder eines pharmazeutisch verträglichen Salzes davon ist.

10. Verfahren nach Anspruch 1 zur Herstellung von 2-[3-(3-(Piperidinomethyl)phenoxy)propyl]-5-aminopyrimidin-4-on oder eines pharmazeutisch verträglichen Salzes davon.

11. Verfahren zur Herstellung einer Verbindung der in Anspruch 1 angegebenen Formel I oder eines Salzes davon, in der $R^4$ ein Wasserstoffatom ist, bei dem man eine Verbindung der Formel XVI

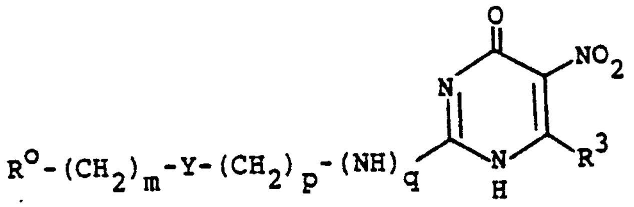

(XVI)

in der $R^o$, m, Y, p, q und $R^3$ die in Anspruch 1 angegebene Bedeutung haben, oder ein Salz davon reduziert.

12. Verfahren zur Herstellung von 2-[3-(3-(Piperidinomethyl)phenoxy)propyl]-5-aminopyrimidin-4-on, bei dem man 2-[3-(3-(Piperidinomethyl)phenoxy)propyl]-5-nitropyrimidin-4-on reduziert.

13. Verfahren nach Anspruch 11 oder 12, bei dem das Reduktionsmittel Zinn(II)-chlorid unter im wesentlichen neutralen Bedingungen enthält.

14. Verfahren nach Anspruch 13, bei dem das Lösungsmittel Äthylacetat enthält und das Zinn(II)-chlorid in wasserfreier Form vorliegt.

15. Verfahren zur Herstellung eines Arzneimittels, bei dem man eine Verbindung der in Anspruch 1 angegebenen Formel I oder ein pharmazeutisch verträgliches Salz davon mit einem pharmazeutisch verträglichen Träger vereinigt.

16. Verfahren nach Anspruch 15, bei dem die Verbindung der Formel I 2-[3-(3-(Piperidinomethyl)-phenoxy)propyl]-5-aminopyrimidin-4-on ist.

**Revendications pour les Etats contractants: BE CH DE FR IT LI LU NL SE**

1. Un composé de formule (I)

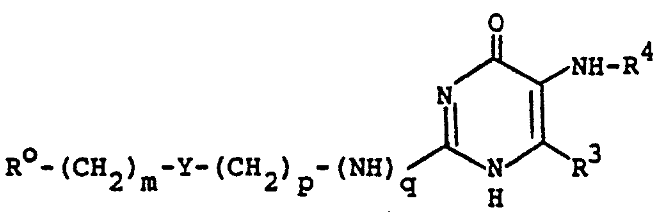

ou un sel de celui-ci, où

$R^o$ est 2-guanidinothiazol-4-yle ou un groupe $R^1R^2N(CH_2)_n$—Z— dans lequel:

$R^1$ et $R^2$ sont indépendamment hydrogène, $C_{1-6}$alkyle, benzyle, phénéthyle, furanyle($C_{1-6}$alkyle), thiényle($C_{1-6}$)alkyle, $C_{3-10}$cycloalkyle, hydroxy($C_{2-6}$)alkyle, ou halo ($C_{2-6}$)alkyle (où les dits groupes hydroxy et halo ne sont pas substitués sur l'atome de carbone adjacent à l'atome d'azote); ou

$R^1$ et $R^2$ ensemble représentent —$(CH_2)^r$— où r est 4 à 7, pour former ensemble avec l'atome d'azote auquel ils sont attachés un noyau saturé de 5 à 8 membres;

n est un entier de 1 à 6;

Z est 2,5-furanyle, 2,5-thiényle, 2,4-pyridyle où le groupe $R^1R^2N(CH_2)_n$ est dans la position 4, 2,4-thiazolyle où le groupe $R^1R^2N(CH_2)_n$ est dans la position 2, ou 1,3- ou 1,4-phénylène;

m est un; ou si Z est pyridyle ou phénylène m peut également être zéro;

Y est oxygène, soufre ou méthylène; ou si Z est furanyle, thiényle ou thiazolyle Y peut également être une liaison;

p est deux, trois ou quatre;

q est zéro ou un;

$R^3$ est hydrogène ou $C_{1-6}$alkyle; et

$R^4$ est hydrogène ou $C_{1-6}$alcanoyle.

2. Un composé selon la revendication 1 que est un composé de formule (I) ou un sel pharmaceutiquement acceptable d'un tel composé.

3. Un composé selon l'une ou l'autre des revendications 1 et 2 dans lequel $R^o$ est un groupe $R^1R^2N(CH_2)_nZ$— dans lequel $R^1$, $R^2$, n et Z sont tels que définis dans la formule (I).

4. Un composé selon l'une quelconque des revendications 1 à 3 dans lequel $R^4$ est hydrogène.

5. Un composé selon l'une quelconque des revendications 1 à 4 dans lequel q est zéro.

6. Un composé selon l'une quelconque des revendications 1 à 4 dans lequel q est un.

7. Un composé selon l'une quelconque des revendications 1 à 6 dans lqeuel $R^3$ est hydrogène.

8. Un composé selon l'une quelconque des revendications 1 à 7 dans lequel $R^1$ et $R^2$ pris ensemble représentent $—(CH_2)^r—$ dans lequel r est 4 à 6.

9. Un composé selon l'une quelconque des revendications 1 à 8 dans lequel $R^1R^2N(CH_2)_n—Z—$ est 3-piperidinométhylphényl.

10. Un composé selon la revendication 2 qui est:

2-[3-(3-(diméthylaminométhyl)phénoxy)propylamino]-5-aminopyrimidine-4-one ou un sel pharmaceutiquement acceptable de ce dernier;

2-[3-(3-(piperidinométhyl)phénoxy)propyl]-5-amino-pyrimidine-4-one ou un sel pharmaceutiquement acceptable de ce dernier;

2-[3-(3-piperidinométhyl)phénoxy)propylamino]-5-acétamidopyrimidine-4-one ou un sel pharmaceutiquement acceptable de ce dernier;

2-[3-(3-(piperidinométhyl)phénoxy)propylamino]-5-aminopyrimidine-4-one ou un sel pharmaceutiquement acceptable de ce dernier;

2-[3-(4-(piperidinométhyl)pyrid-2-oxy)propylamino]-5-aminopyrimidine-4-one ou un sel pharmaceutiquement acceptable de ce dernier;

2-[3-(4-piperidinométhyl)pyrid-2-oxy)propyl]-5-aminopyrimidine-4-one ou un sel pharmaceutiquement acceptable de ce dernier, ou

2-[3-[3-(hexahydroazépinométhyl)phénoxy]propylamino]-5-aminpyrimidine-4-one ou un sel pharmaceutiquement acceptable de ce dernier.

11. Un composé selon la revendication 2 qui est 2-[3-(3-(piperidinométhyl)phénoxy)propyl]-5-amino-pyrimidine-4-one ou un sel pharmaceutiquement acceptable de ce dernier.

12. Dihydrochlorure de 2-[3-(3-(piperidinométhyl)phénoxy)propyl]-2-amino-pyrimidin-4-one.

13. Une composition pharmaceutiquement comprenant un composé selon l'une quelconque des revendications 2 à 12 et un véhicule pharmaceutiquement acceptable.

14. Un composé selon l'une quelconque des revendications 2 à 12 pour emploi comme agent thérapeutique.

15. Un composé selon l'une quelconque des revendications 2 à 12 pour emploi comme antagoniste d'histamine $H_2$.

16. Un procédé pour la préparation d'un composé de formule (I) tel que défini dans la revendication 1 ou un sel dudit composé, lequel procédé prévoit:

a) dans le cas de composés de la formule (I) dans lesquels q est un, réaction d'un composé de la formule (II) avec un composé de la formule (III):

$$R^6(CH_2)_m\text{-}Y\text{-}(CH_2)_pNH_2$$

(II)

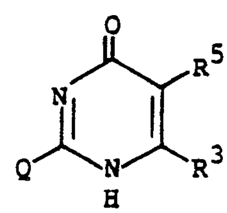

(III)

dans lequel m, Y, p et $R^3$ sont tels que définis ci-avant, $R^6$ est un groupe $R^o$ tel que défini ci-avant ou $R^6$ est un groupe furane-2-yle ou thiène-2-yle; $R^5$ est amino protégé; et Q est un groupe qui peut être déplacé par une amine; ou

b) dans le cas de composés de la formule (I) dans lesquels m est un et Y est soufre, réaction d'un composé de la formule (IV) avec un composé de la formule (V):—

$$R^6\text{-}CH_2\text{-}L$$

(IV)

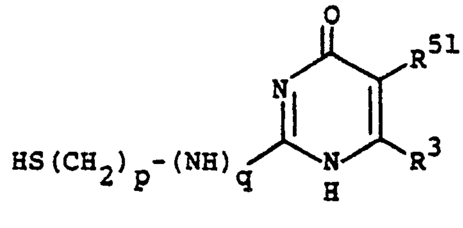

(V)

où $R^6$, p, q et $R^3$ sont tels que définis ci-avant, $R^{51}$ est amino optionnellement protégé et L est un groupement déplaçable par un thiol ou un équivalent chimique de ce dernier; ou

c) dans le cas de composés de la formule (I) dans lesquels m est un et Y est soufre, réaction d'un composé de la formule (VI) ou équivalent chimique de celui-ci avec un composé de la formule (VII):

$R^6CH_2SH$

(VI)

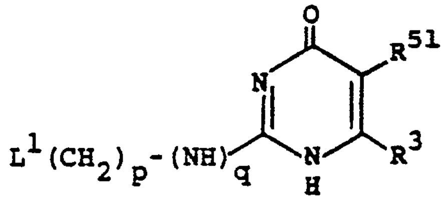

(VII)

où $R^6$, p, q, $R^{51}$ et $R^3$ sont tels que définis ci-avant et $L^1$ est un groupement deplaçable par thiol ou un groupement chimiquement équivalent; ou

d) réaction d'un composé de la formule (VIII) avec un composé de la formule (IX) ou un équivalent chimique de ce dernier:

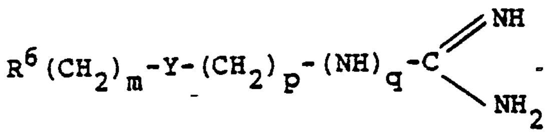

(VIII)

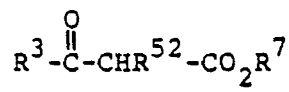

(IX)

où $R^6$, m, Y, p, q est $R^3$ sont tels que définis ci-avat, $R^{52}$ est amino protégé et $R^7$ est un groupe formant un ester; ou

e) dans le cas de composés de la formule (I) dans lesquels Z est 2,4-pyridyle, m est zéo et Y est oxygène, réaction d'un composé de formule (X) avec un composé de formule (XI) ou un dérivé de ce dernier susceptible de produire une réaction:

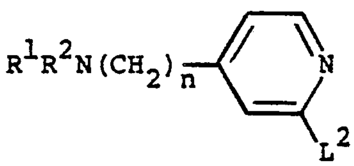

(X)

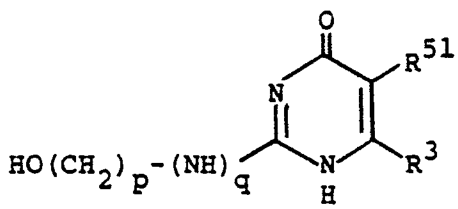

(XI)

où $R^1$, $R^2$, n, p, q, $R^3$ et $R^{51}$ sont tels que définis ci-avant et $L^2$ est un groupe déplaçable par hydroxy ou un groupe chimiquement équivalent; ou

f) dans le cas de composés de formule (I) dans lesquels Z est phénylène, m est zéro et Y est oxygène, réaction d'un composé de la formule (XII) ou d'un équivalent chimique avec un composé de formule (XIII):

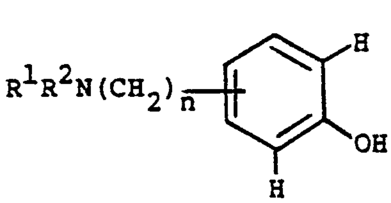

(XII)

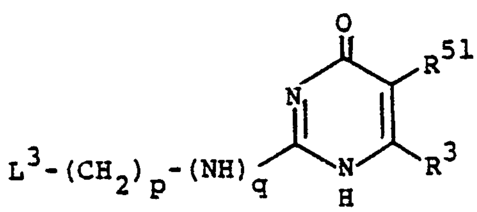

(XIII)

où $R^1$, $R^2$, n, p, q, $R^3$ et $R^{51}$ sont tels que définis ci-avant et $L^3$ est un groupement chimiquement équivalent; ou

g) dans le cas de composés de formule (I) dans lesquels $R^o$ est $R^1R^2N(CH_2)_n$—Z—, conversion du composé de la formule (XIV)

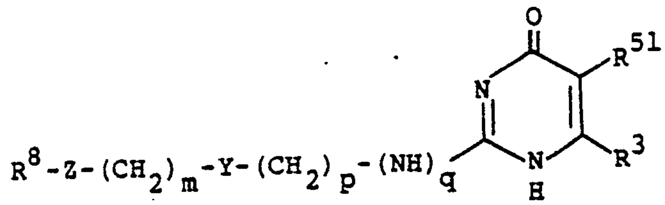

(XIV)

dans lequel Z, m, Y, p, q, $R^3$ et $R^5$ sont tels que définis ci-avant et $R^8$ est un précurseur d'un groupe $R^1R^2N(CH_2)_n$ tel que défini ci-avant; ou

h) dans le cas de composés de formule (I) dans lesquels q est un, réduction d'un composé de formule (XV):

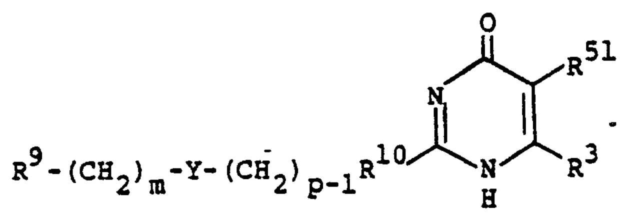

(XV)

où m, Y, p, $R^3$ et $R^{51}$ sont tels que définis ci-avant, $R^9$ est un groupe $R^\circ$ ou $R^8$—Z— tel que défini si avant et $R^{10}$ est un groupe —CH=N— ou —CO—NH—;

et par conséquent, lorsque cela est nécessaire:

i) réaction d'un composé dans lequel $R^6$ est furane-2-yle ou thiène-2-yle avec un réactif de Mannich pour former un composé de formule (I) dans lequel n est un;

ii) conversion d'un groupe amino protégé en amino ou $C_{1-6}$alcanoylamino)

iii) formation optionnelle d'un sel.

17. Un procédé pour la préparation d'un composé de formule (I) tel que défini dans la revendication 1 ou d'un sel dudit composé où $R^4$ est hydrogène, ce procédé faisant appel à la réduction du composé de formule (XVI) ou d'un sel de celui-ci:

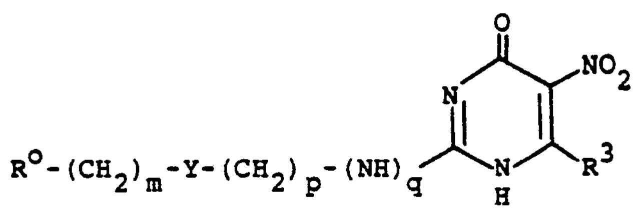

(XVI)

où $R^\circ$, m, Y, p, q et $R^3$ sont tels que définis dans la revendication 1.

18. Un procédé pour la préparation de 2-[3-(3-(piperidinométhyl)phénoxy)-propyl]-5-aminopyrimidine-4-one prévoyant la réduction de 2-[3-(3-(piperidinométhyl)phénoxy)propyl-5-nitropyrimidine-4-one.

19. Un processus selon la revendication 17 ou 18 dans lequel l'agent réducteur comprend du chlorure stanneux.

20. Un procédé selon la revendication 19 dans lequel le solvant comprend de l'acétate d'éthyle et du chlorure stanneux sous sa forme anhydre.

21. Un composé de formule (XVI):

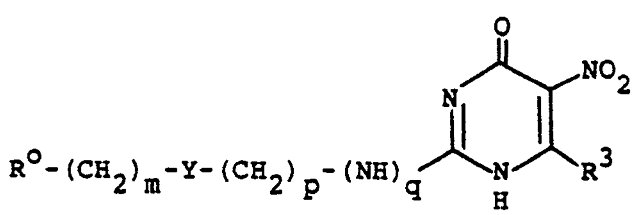

(XVI)

ou un sel de celui-ci dans lequel $R^\circ$, m, Y, p, q et $R^3$ sont tels que définis dans la revendication 1.

22. 2-[3-(3-(piperidinométhyl)phénoxy)propyl]-5-nitropyrimidine-4-one.

**Revendications pour l'Etat contractant: AT**

1. Un procédé pour la préparation d'un composé de formule (I):

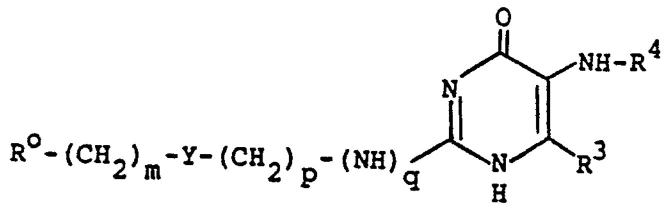

(I)

ou un sel d'un tel composé, où:

$R^o$ est 2-guanidinothiazol-4-yle ou un groupe $R^1R^2N(CH_2)_n$—Z— dans lequel:

$R^1$ et $R^2$ sont indépendamment hydrogène, $C_{1-6}$alkyle, benzyle, phénéthyle, furanyle($C_{1-6}$)alkyle, thiényle($C_{1-6}$)alkyle, $C_{3-10}$cycloalkyle, hydroxy($C_{2-6}$)alkyle, ou halo ($C_{2-6}$)alkyle (où les groupes hydroxy et halo ne sont pas substitués sur l'atome de carbone adjacent à l'atome d'azote); ou

$R^1$ et $R^2$ ensemble représentent —$(CH_2)^r$— où r est 4 à 7, pour former ensemble avec l'atome d'azote auquel ils sont attachés un noyau saturé de 5 à 8 membres;

n est entier de 1 à 6;

Z est 2,5-furanyle, 2,5-thiényle, 2,4-pyridyle où le groupe $R^1R^2N(CH_2)_n$ est à la position 4, 2,4-thiazolyle où le groupe $R^1R^2N(CH_2)_n$ est à la position 2, ou 1,3- ou 1,4-phénylène;

m est un; ou si Z est pyridyle ou phénylène m peut également être zéro;

Y est oxygène, soufre ou méthylène; ou si Z est furanyle, thiényle ou thiazolyle Y peut également être une liaison;

p est deux, trois ou quatre;

q est zéro ou un;

$R^3$ est hydrogène ou $C_{1-6}$alkyle; et

$R^4$ est hydrogène ou $C_{1-6}$alcanoyle:

lequel procédé prévoit:

a) dans le cas de composés de la formule (I) où q est un, réaction d'un composé de la formule (II) avec un composé de la formule (III):—

$$R^6(CH_2)_m\text{-}Y\text{-}(CH_2)_pNH_2$$

(II)

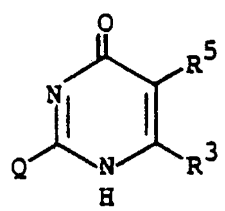

(III)

où m, Y, p et $R^3$ sont tels que définis ci-avant, $R^6$ est un groupe $R^o$ tel que défini ci-avant ou $R^6$ est un groupe furane-2-yle ou thiène-2-yle; $R^5$ est amino protégé; et Q est un groupe pouvant être déplacé par une amine; ou

b) dans le cas de composés de formule (I) où m est un et Y est soufre, réaction d'un composé de formule (IV) avec un composé de formule (V)

$$R^6\text{-}CH_2\text{-}L$$

(IV)

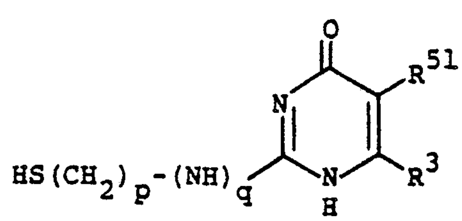

(V)

où $R^6$, p, q et $R^3$ sont tels que définis ci-avant, $R^{51}$ est optionnellement amino protégé et L est un groupement susceptible d'être déplacé par un thiol ou un groupement chimiquement équivalent; ou

c) dans le cas de composés de formule (I) dans lesquels m est un et Y est soufre, réaction d'un composé de formule (VI) ou d'un équivalent chimique avec un comosé de formule (VII):

$R^6CH_2SH$

(VI)

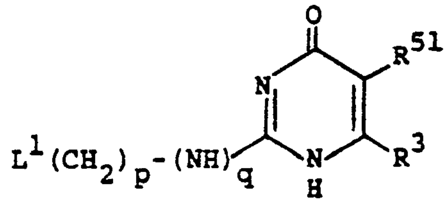

(VII)

où $R^6$, p, q, $R^{51}$ et $R^3$ sont tels que définis ci-avant et $L^1$ est un groupement deplaçable par un thiol ou un groupement chimiquement équivalent; ou

d) réaction d'un composé de la formule (VIII) avec un composé de la formule (IX) ou un équivalent chimique:

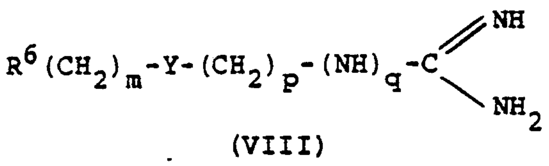

(VIII)

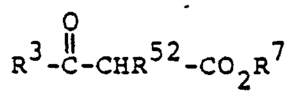

(IX)

où $R^6$, m, Y, p, q est $R^3$ sont tels que définis ci-avant, $R^{52}$ est amino protégé et $R^7$ est un groupe formant un ester; ou

e) dans le cas de composés de formule (I) où Z est 2,4-pyridyle, m est zéro et Y est oxygène, réaction d'un composé de formule (X) avec un composé de formule (XI) ou un dérivé d'un tel composé permettant la réaction de se produire:

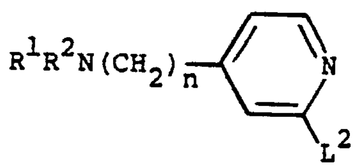

(X)

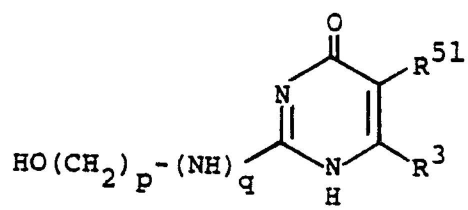

(XI)

où $R^1$, $R^2$, n, p, q, $R^3$ et $R^{51}$ sont tels que définis ci-avant et $L^2$ est un groupe déplaçable par hydroxy ou un groupe équivalent; ou

f) dans le cas de composés de formule (I) dans lesquels Z est phénylène, m est zéro et Y est oxygène, réaction d'un composé de formule (XII) ou d'un équivalent chimique avec un composé de formule (XIII):

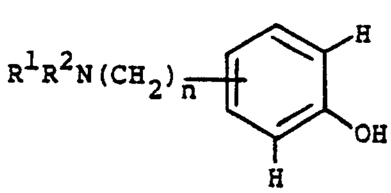

(XII)

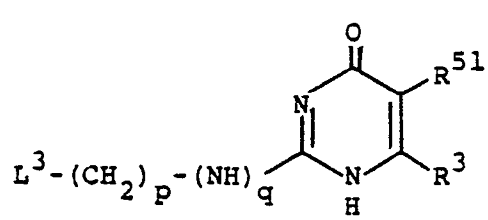

(XIII)

où $R^1$, $R^2$, n, p, q, $R^3$ et $R^{51}$ sont tels que définis ci-avant et $L^3$ est un groupement déplaçable par phénol ou un groupement chimiquement équivalent; ou

g) dans le cas de composés de formule (I) dans lesquels $R^o$ est $R^1R^2N(CH_2)_n$—Z—, conversion du composé de formule (XIV)

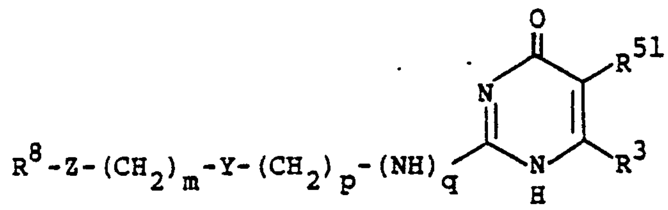

(XIV)

où Z, m, Y, p, q, $R^3$ et $R^5$ sont tels que définis ci-avant et $R^8$ est un précurseur d'un groupe $R^1R^2N(CH_2)_n$ tel que défini ci-avant; ou

h) dans le cas de composés de formule (I) où q est un, réduction d'un composé de formule (XV):

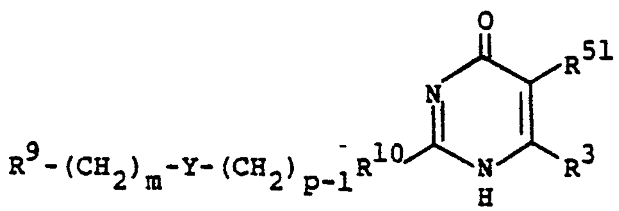

(XV)

où m, Y, p, $R^3$ et $R^{51}$ sont tels que définis ci-avant, $R^9$ est un groupe $R^o$ ou $R^8$—Z— tel que défini si avant et $R^{10}$ est un groupe —CH=N— ou —CO—NH—;

et par la suite, lorsque cela sera nécessaire:

i) réaction d'un composé dans lequel $R^6$ est furan-2-yle ou thiène-2-yle avec un réactif de Mannich pour former un composé de formule (I) dans lequel n est un;

ii) conversion d'un groupe amino protégé en un groupe amino ou $C_{1-6}$alcanoylamino)

iii) formation optionnelle d'un sel.

2. Un procédé selon la revendication 1 pour la préparation d'un composé de formule (I) ou d'un sel dudit composé où $R^o$ est un groupe $R^1R^2N(CH_2)_nZ$ dans lequel $R^1$, $R^2$, n et Z sont tels que définis dans la formule (I) lorsque le procédé est exécuté selon l'une quelconque des variantes a) à d).

3. Un procédé selon l'une quelconque des revendications 1 ou 2 pour la préparation d'un composé de formule (I) ou d'un sel de celui-ci où $R^4$ est hydrogène.

4. Un procédé selon l'une quelconque des revendications 1 à 3 pour la préparation d'un composé de formule (I) ou d'un sel de celui-ci où q est zèro.

5. Un procédé selon l'une quelconque des revendications 1 à 3 pour la préparation d'un composé de formule (I) ou d'un sel de celui-ci où q est un.

6. Un procédé selon l'une quelconque des revendications 1 à 5 pour la préparation d'un composé de formule (I) ou d'un sel de celui-ci où $R^3$ est hydrogène.

7. Un procédé selon l'une quelconque des revendications 1 à 6 pour la préparation d'un composé de formule (I) ou d'un sel de celui-ci où $R^1$ et $R^2$ ensemble représentent $—(CH_2)_r$ où r est 4 à 6.

8. Un procédé selon l'une quelconque des revendications 1 à 7 pour la préparation d'un composé de formule (I) ou d'un sel de celui-ci où $R^1R^2N(CH_2)_n$—Z— est 3-piperidinométhylphényle-.

9. Un procédé selon la revendication 1 pour la préparation de:

2-[3-(3-(diméthylaminométhyl)phénoxy)propylamino]-5-aminopyrimidine-4-one ou un sel pharmaceutiquement acceptable de celui-ci,

2-[3-(3-(piperidinométhyl)phénoxy)propyl]-5-amino-pyrimidine-4-one ou un sel pharmaceutiquement acceptable de celui-ci,

2-[3-(3-piperidinométhyl)phénoxy)propylamino]-5-acétamidopyrimidine-4-one ou un sel pharmaceutiquement acceptable de celui-ci,

2-[3-(3-(piperidinométhyl)phénoxy)propylamino]-5-aminopyrimidine-4-one ou un sel pharmaceutiquement acceptable de celui-ci,

2-[3-(4-(piperidinométhyl)pyrid-2-oxy)propylamino]-5-aminopyrimidine-4-one ou un sel pharmaceutiquement acceptable de celui-ci,

2-[3-(4-piperidinométhyl)pyrid-2-oxy)propyl]-5-aminopyrimidine-4-one ou un sel pharmaceutiquement acceptable de celui-ci, ou

2-[3-[3-(hexahydroazépinométhyl)phénoxy]propylamino]-5-aminopyrimidine-4-one ou un sel pharmaceutiquement acceptable de celui-ci.

10. Un procédé selon la revendication 1 pour la préparation de 2-[3-(3-(piperidinométhyl)phénoxy)-propyl]-5-aminopyrimidine-4-one ou un sel pharmaceutiquement acceptable de celui-ci.

11. Un procédé pour la préparation d'un composé de formule (I) tel que défini dans la revendication 1 ou d'un sel de ce composé où $R^4$ est hydrogène, porocédé prévoyant la réduction d'un composé de formule (XVI) ou d'un sel de celui-ci:

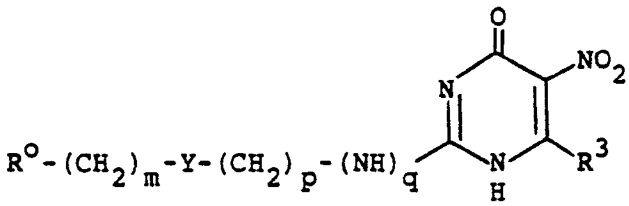

(XVI)

où R°, m, Y, p, q et $R^3$ sont tels que définis dans la revendication 1.

12. Un procédé pour la préparation de 2-[3-(3-(piperidinomèthyl)phénoxy)propyl]-5-aminopyrimidine-4-one prévoyant la réduction de 2-[3-(3-(piperidinométhyl)phénoxy)propyl]-5-nitropyrimidine-4-one.

13. Un procédé selon l'une ou l'autre de revendication 11 ou 12 dans lequel l'agent réducteur comprend du chlorure stanneux sous des conditions essentiellement neutres.

14. Un procédé selon la revendication 13 dans lequel le solvant comprend de l'acétate d'éthyle, et le chlorure stanneux est sous forme anhydre.

15. Un processus pour la préparation d'une composition pharmaceutiquement prévoyant l'association d'un composé de formule (I) tel que défini dans la revendication 1 ou d'un sel pharmaceutiquement acceptable dudit composé et d'un véhicule pharmaceutiquement acceptable.

16. Un procédé selon la revendication 15 dans lequel le composé de formule (I) est 2-[3-(3-(piperidinométhyl)phénoxy)propyl]-5-aminopyrimidine-4-one.